# EUROPEAN PATENT APPLICATION

(11) **EP 4 393 511 A1**
(43) Date of publication of application: **03.07.2024**
(21) Application number: 23838764.1
(22) Date of filing: 30.06.2023
(51) Int. Cl.: A61K 47/16, C07C 69/40, A61K 47/12, A61K 47/06, A61K 9/127, A61K 31/7105

(54) **LIPID COMPOUND AND USE THEREOF**

(30) Priority: 11.07.2022 CN 202210809436
(71) Applicant: Themedium Therapeutics Co., Ltd., Suzhou, Jiangsu 215011 (CN)
(72) Inventor: ZHANG, Lei, Suzhou, Jiangsu 215011 (CN); CHEN, Jianxin, Suzhou, Jiangsu 215011 (CN); YAN, Lu, Suzhou, Jiangsu 215011 (CN); PENG, Wei, Suzhou, Jiangsu 215011 (CN); MA, Qiaoqiao, Suzhou, Jiangsu 215011 (CN)
(74) Representative: LLR
(86) International application number: PCT/CN2023/104925
(87) International publication number: WO 2024/012272

(57) **Abstract**

The present application provides a novel lipid compound, a lipid nanoparticle comprising the same, and use thereof in targeted delivery of a drug. Specifically, the present application provides a compound of Formula I 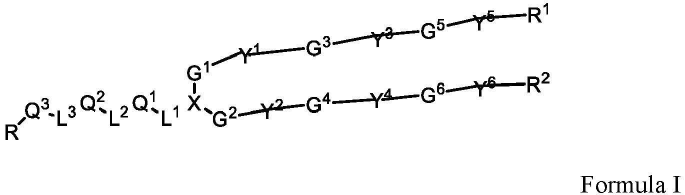 or a pharmaceutically acceptable salt, a prodrug, or a stereoisomer thereof. Furthermore, the present application provides a lipid nanoparticle comprising the compound of Formula I or a pharmaceutically acceptable salt, a prodrug, or a stereoisomer thereof, still further, the lipid nanoparticle may further comprise a cationic lipid; yet still further, the lipid nanoparticle may further comprise a helper lipid. A composition comprising the above compound or a pharmaceutically acceptable salt, a prodrug, or a stereoisomer thereof, or the lipid nanoparticle can achieve the organ-targeted delivery of a therapeutic/prophylactic agent.

## Description

### CROSS-REFERENCE

This application claims the priority of the Chinese patent application 202210809436.X filed on July 11,2022 with the invention name "Composition for Organ-specific Delivery of Nucleic Acids" , and the full contents of which are incorporated herein by reference.

### TECHNICAL FIELD

The present disclosure relates,,generally to the field of chemistry, and particularly to a novel ionizable anionic lipid and a pharmaceutically acceptable salt, a prodrug, or a stereoisomer thereof, a nanoparticle composition comprising the lipid, and use of the nanoparticle composition in organ-specific delivery of substances such as a nucleic acid.

### BACKGROUND

Therapeutic or prophylactic nucleic acids have the potential to revolutionize vaccination, gene therapy, protein alternative therapy and other therapies for genetic diseases. Since the first clinical study on therapeutic nucleic acids in the 2000s, significant advances have been made in the research on the design of nucleic acid molecules and the method of delivering them. However, nucleic acid drugs (including therapeutic and prophylactic drugs) still face several challenges, including the accumulation of most liposome formulations through biological processes in the liver, thereby reducing the efficacy of compositions delivered into target organs. Similarly, other therapeutic agents such as proteins and small molecule drugs can benefit from organ-specific delivery. Many different types of compounds such as chemical drugs exhibit significant cytotoxicity. If these compounds could be delivered to the desired organ on-target, there would be fewer off-target and side effects.

Currently, most lipid nanoparticle delivery systems passively target at liver. A common strategy to alter the target organ of a lipid nanoparticle is to adjust the composition of the lipid nanoparticle. According to some reports, lipid nanoparticles composed of 1-2 types of lipids can achieve the purpose of targeting the spleen by adjusting the ratio in which the nucleic acid and the nanoparticles are mixed (Stephan Grabbe et al., Translating Nanoparticulate-personalized Cancer Vaccines into Clinical Applications: Case Study with RNA-Lipoplexes for the Treatment of Melanoma, 2016); however, this strategy needs improvement to stability of formulation, encapsulation efficiency, etc. In addition, according to some reports (Cheng Qiang et al., Selective organ targeting (SORT) nanoparticles for tissue-specific mRNA delivery and CRISPR-Cas gene editing, 2020), adding permanently anionic lipids to LNPs consisting of four components-a cationizable lipid, a steroid, a phospholipid, and a PEG lipid-also enables the specific targeting of the spleen, however which will increase the complexity of the components and the formulation process. Therefore, a lipid nanoparticle delivery system with high delivery capacity, high stability and low complexity remains to be developed.

### SUMMARY

The present disclosure provides a compound of the following Formula I or a pharmaceutically acceptable salt, a prodrug, a stereoisomer, or a deuteride thereof: 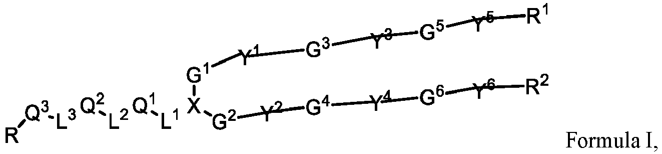
wherein L¹ is absent, -C-C-, -C=C-, -C≡C-, -O(C=O)-, -(C=O)O-, -C(=O)-, -O(C=O)O-, -O-, -S(O)ₓ-, -S-S-, - C(=O)S-, -SC(=O)-, -NR^{a}C(=O)-, -C(=O)NR^{a}-, -NR^{a}C(=O)NR^{a}-, -OC(=O)NR^{a}-, -NR^{a}C(=O)O-, -NR^{a}(CH₂)ₓ-, or -(CH₂)ₓNR^{a}-; Q¹ is absent or C1-C8 linear or branched hydrocarbyl, and specifically can be C1, C2, C3, C4, C5, C6, C7, or C8 linear or branched hydrocarbyl;
L² is absent, -C-C-, -C=C-, -C≡C-, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-; Q² is absent or C1-C8 linear or branched hydrocarbyl, and specifically can be C1, C2, C3, C4, C5, C6, C7, or C8 linear or branched hydrocarbyl;
L³ is absent, -C-C-, -C=C-, -C≡C-, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-; Q³ is absent or C1-C8 linear or branched hydrocarbyl, and specifically can be C1, C2, C3, C4, C5, C6, C7, or C8 linear or branched hydrocarbyl;
R^{a} is H, deuterium, C1-C8 linear or branched hydrocarbyl, or C1-C8 linear or branched hydrocarbyl substituted with carboxyl, and specifically can be C1, C2, C3, C4, C5, C6, C7, or C8 linear or branched hydrocarbyl;
X is C or N;
R is 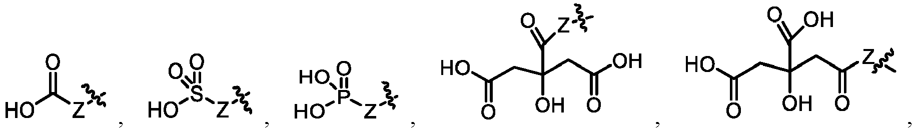 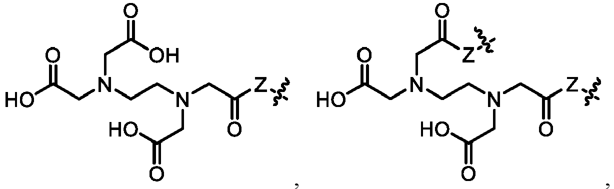 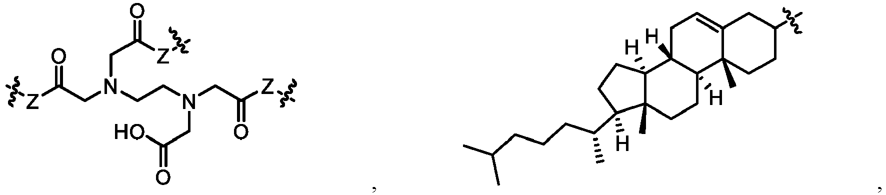 or 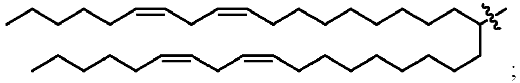 Z is C, O, NR^{b}, or S; R^{b} is H, deuterium, or C1-C8 linear or branched hydrocarbyl, and specifically can be C1, C2, C3, C4, C5, C6, C7, or C8 linear or branched hydrocarbyl;
one of Y¹ and Y² is absent, -C-C-, -C=C-, -C≡C-, -O(C=O)-, -C(OH)-, -(C=O)O-, -C(=O)-, -O(C=O)O-, -O-, - S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-, and the other one of Y¹ and Y² is absent, -C-C-, -C=C-, -C≡C-, -O(C=O)-, -C(OH)-, -(C=O)O-, - C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, - OC(=O)NR^{a}-, or -NR^{a}C(=O)O-;
G¹ and G² are each independently absent or substituted C1-C12 linear or branched hydrocarbyl, and specifically can be C1, C2, C3, C4, C5, C6, C7, C8, C9, C10, C11, or C12 linear or branched hydrocarbyl;
one of Y³ and Y⁴ is absent, -C-C-, -C=C-, -C≡C-, -O(C=O)-, -C(OH)-, -(C=O)O-, -C(=O)-, -O(C=O)O-, -O-, - S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-, and the other one of Y³ and Y⁴ is absent, -C-C-, -C=C-, -C≡C-, -O(C=O)-, -C(OH)-, -(C=O)O-, - C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, - OC(=O)NR^{a}-, or -NR^{a}C(=O)O-;
G³ and G⁴ are each independently absent or substituted C1-C12 linear or branched hydrocarbyl, and specifically can be C1, C2, C3, C4, C5, C6, C7, C8, C9, C10, C11, or C12 linear or branched hydrocarbyl;
one of Y⁵ and Y⁶ is absent, -C-C-, -C=C-, -C≡C-, -O(C=O)-, -C(OH)-, -(C=O)O-, -C(=O)-, -O(C=O)O-, -O-, - S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-, and the other one of Y⁵ and Y⁶ is absent, -C-C-, -C=C-, -C≡C-, -O(C=O)-, -C(OH)-, -(C=O)O-, - C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, - OC(=O)NR^{a}-, or -NR^{a}C(=O)O-;
R^{a} is H, deuterium, C1-C8 linear or branched hydrocarbyl, or C1-C8 linear or branched hydrocarbyl substituted with carboxyl, and specifically can be C1, C2, C3, C4, C5, C6, C7, or C8 linear or branched hydrocarbyl;
G⁵ and G⁶ are each independently absent or substituted C1-C12 linear or branched hydrocarbyl, and specifically can be C1, C2, C3, C4, C5, C6, C7, C8, C9, C10, C11, or C12 linear or branched hydrocarbyl;
R¹ and R² are each independently substituted C1-C24 linear or branched hydrocarbyl, and specifically can be C1, C2, C3, C4, C5, C6, C7, C8, C9, C10, C11, C12, C13, C14, C15, C16, C17, C18, C19, C20, C21, C22, C23, or C24 linear or branched hydrocarbyl;
x is 0, 1, or 2.

According to Formula I described above, the present disclosure provides specific lipid compounds as shown in Table 1.

**Table 1. Representative structures of lipid compounds**

| No. | Structural formula |
|---|---|
| 40 | 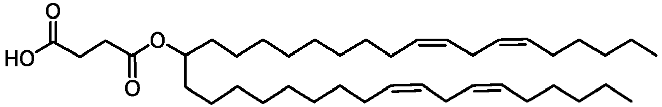 |
| 41 | 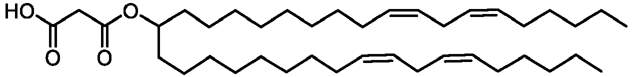 |
| 42 | 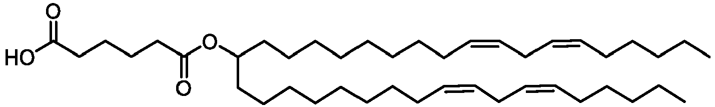 |
| 43 | 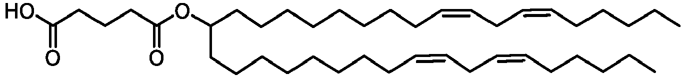 |
| 44 | 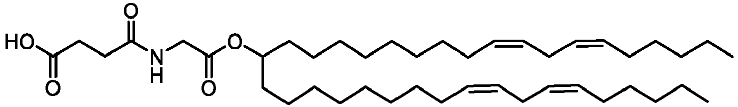 |
| 45 | 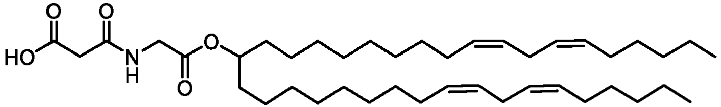 |
| 46 | 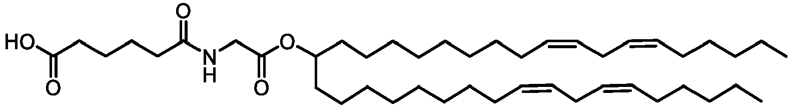 |
| 47 | 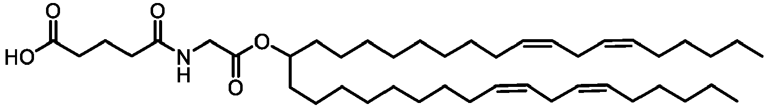 |
| 48 | 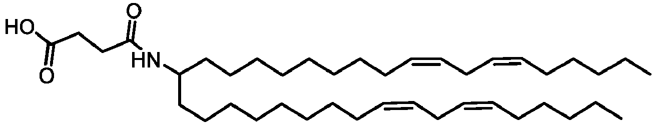 |
| 49 | 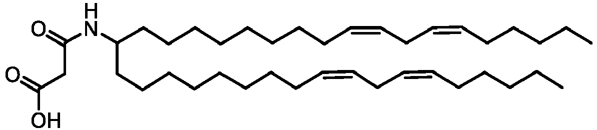 |
| 50 | 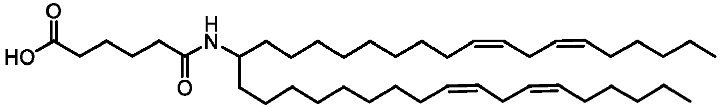 |
| 51 | 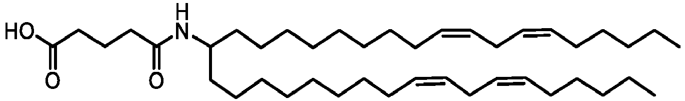 |
| 52 | 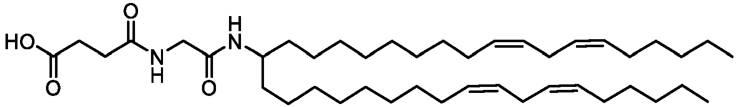 |
| 53 | 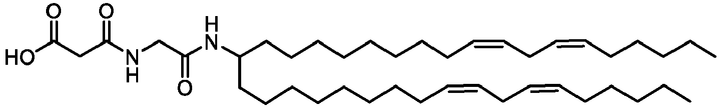 |
| 54 | 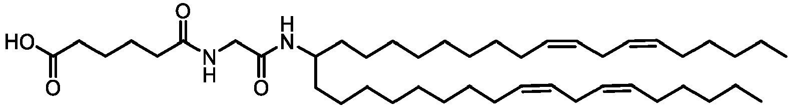 |
| 55 | 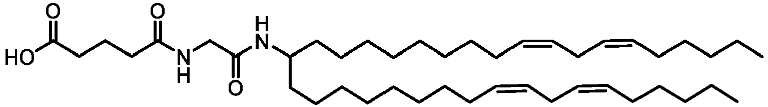 |
| 56 | 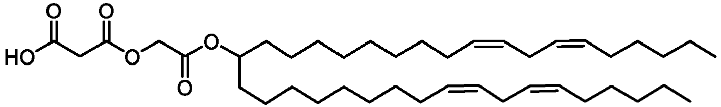 |
| 57 | 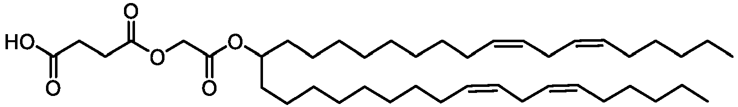 |
| 58 | 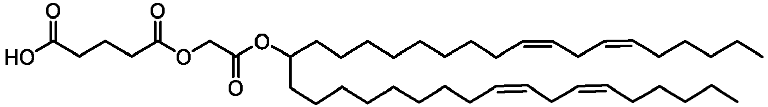 |
| 59 | 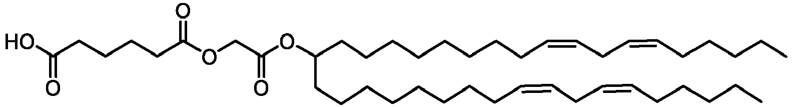 |
| 60 | 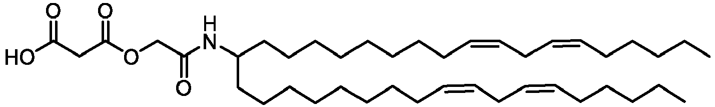 |
| 61 | 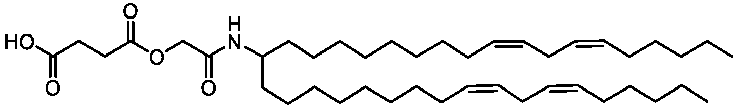 |
| 62 | 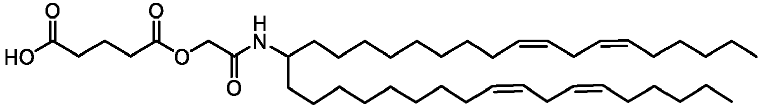 |
| 63 | 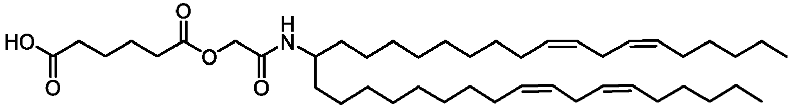 |
| 64 | 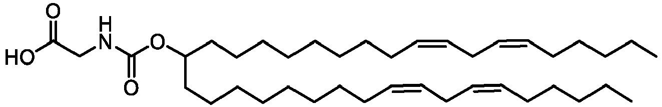 |
| 65 | 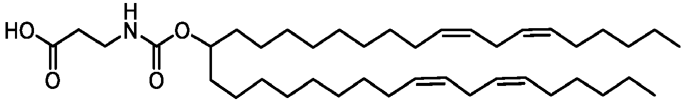 |
| 66 | 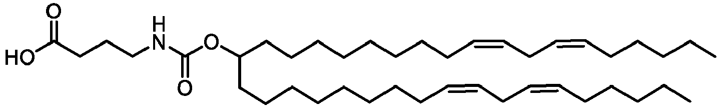 |
| 67 | 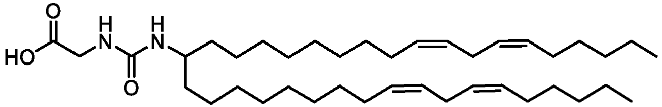 |
| 68 | 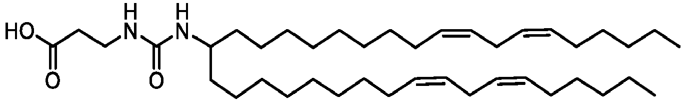 |
| 69 | 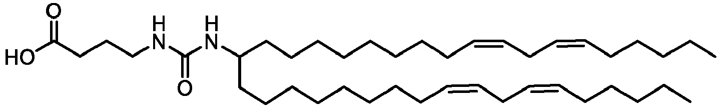 |
| 70 | 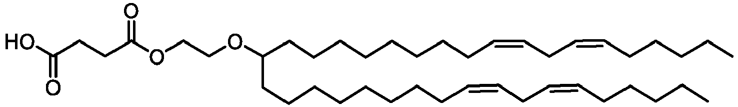 |
| 71 | 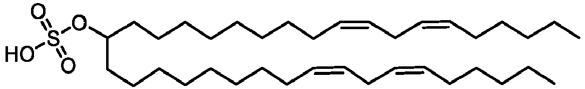 |
| 72 | 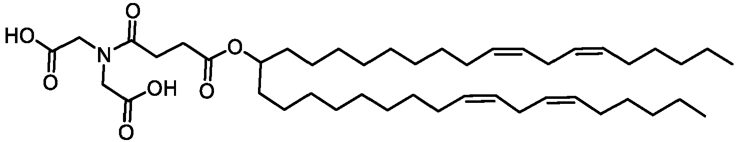 |
| 73 | 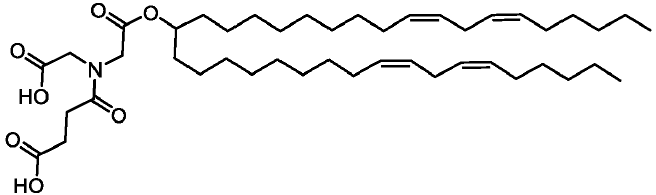 |
| 74 | 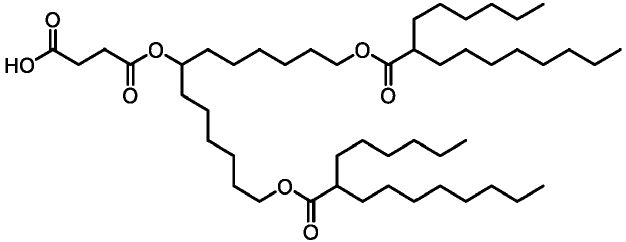 |
| 75 | 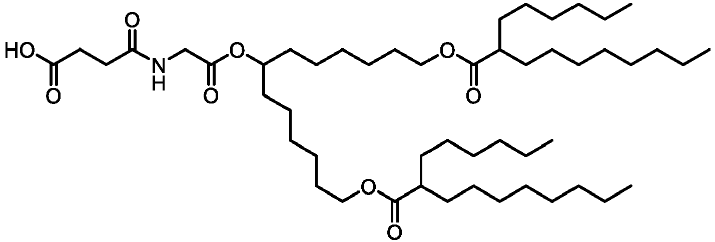 |
| 76 | 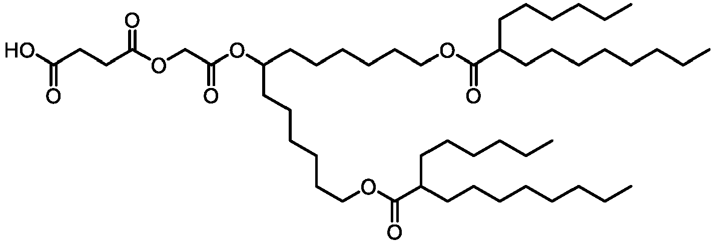 |
| 77 | 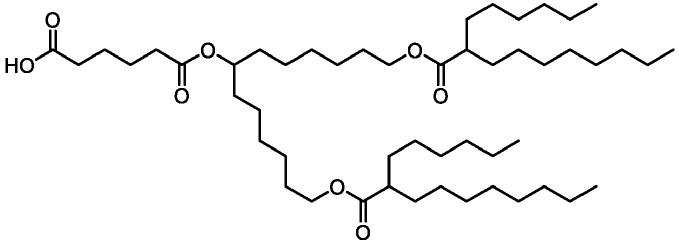 |
| 78 | 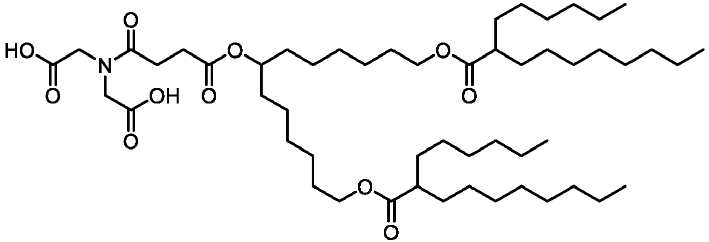 |
| 79 | 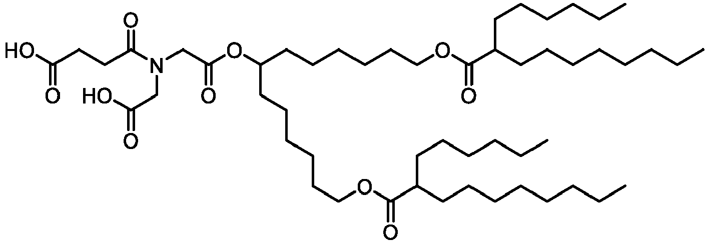 |
| 80 | 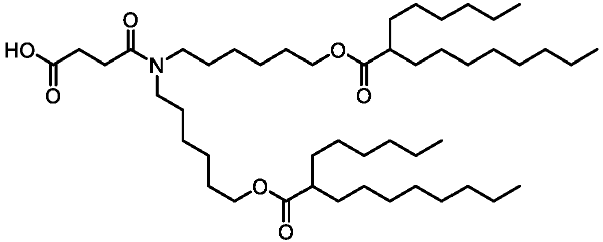 |
| 81 | 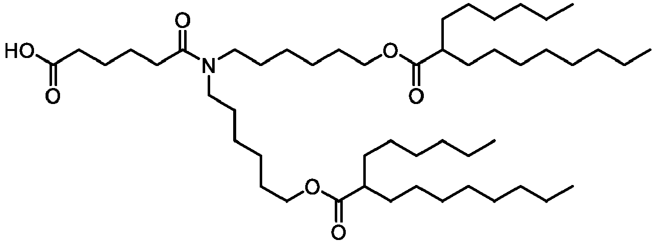 |
| 82 | 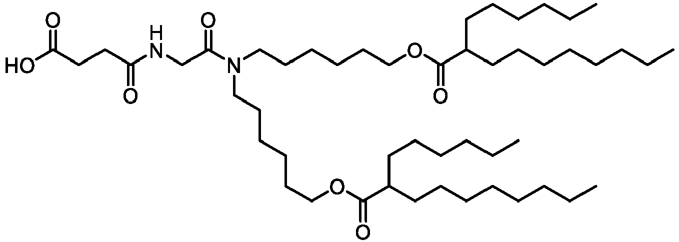 |
| 83 | 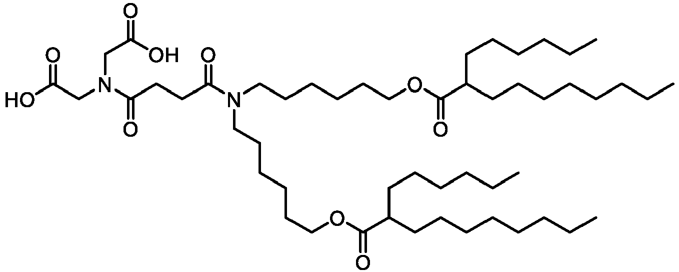 |
| 84 | 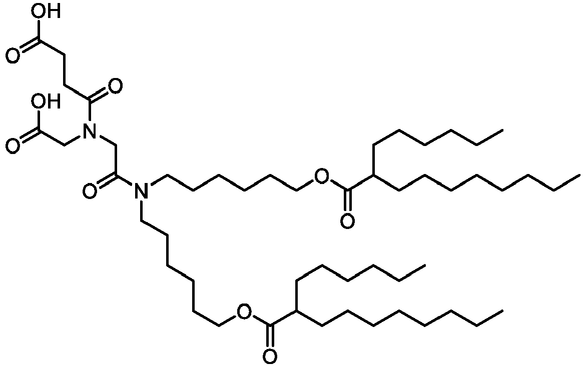 |
| 85 | 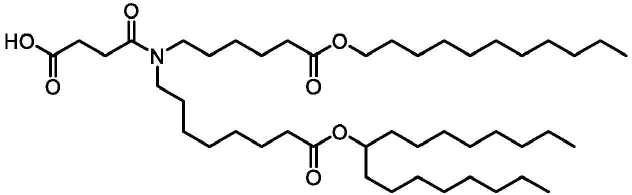 |
| 86 | 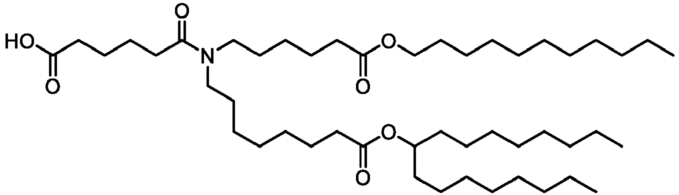 |
| 87 | 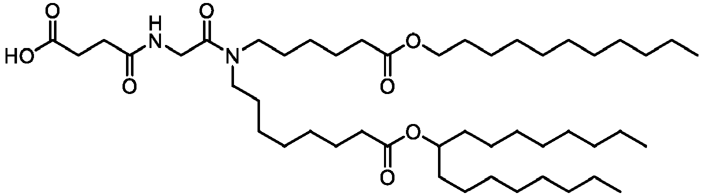 |
| 88 | 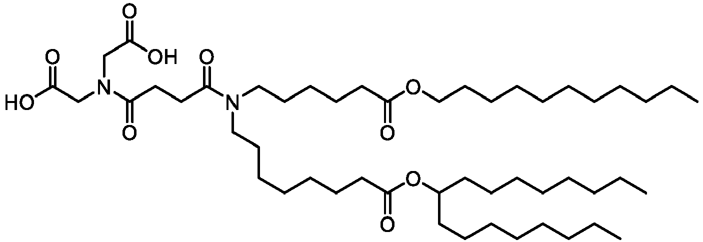 |
| 89 | 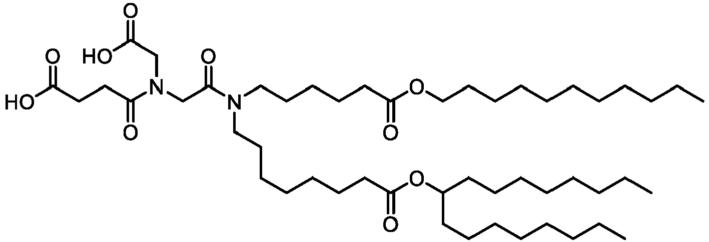 |
| 90 | 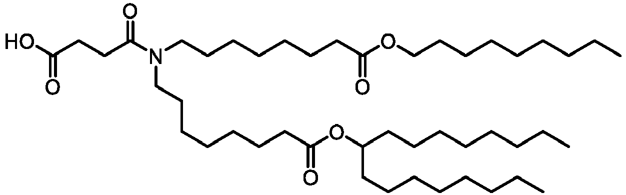 |
| 91 | 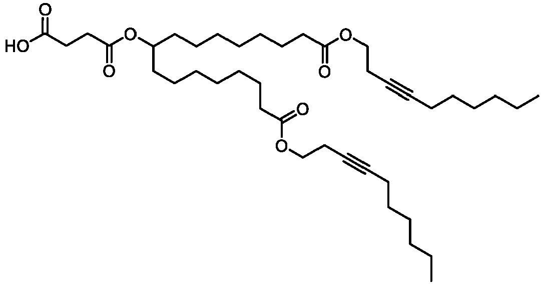 |
| 92 | 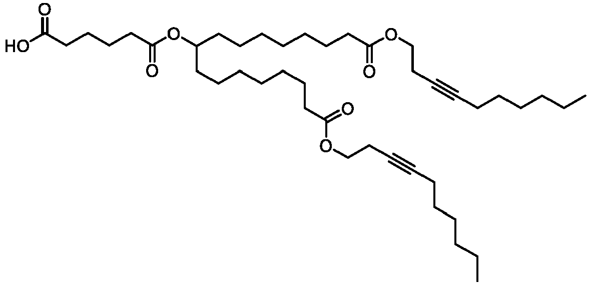 |
| 93 | 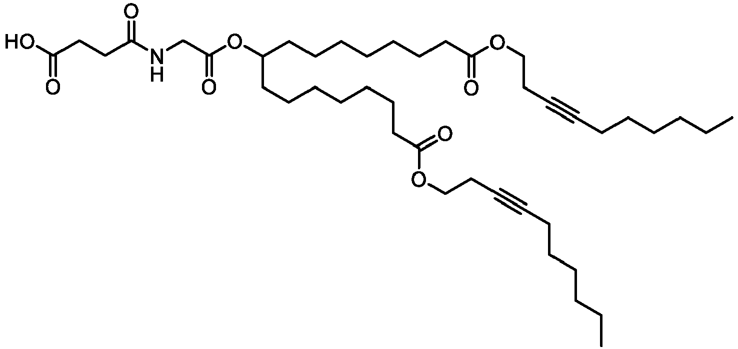 |
| 94 | 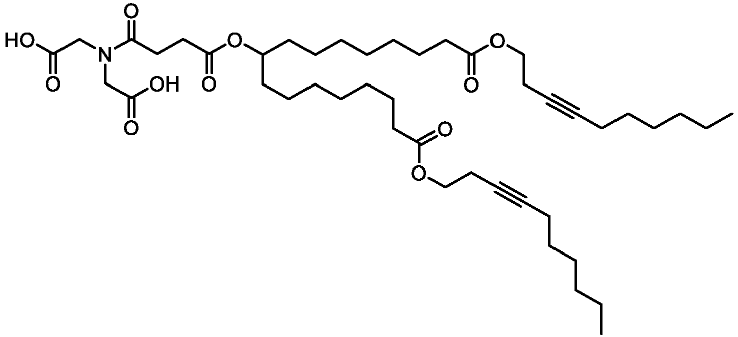 |
| 95 | 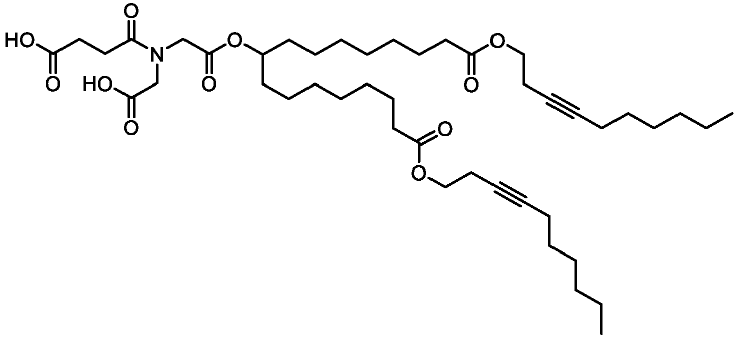 |
| 96 | 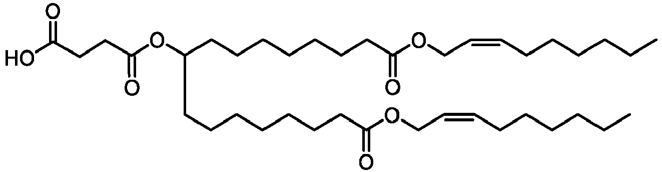 |
| 97 | 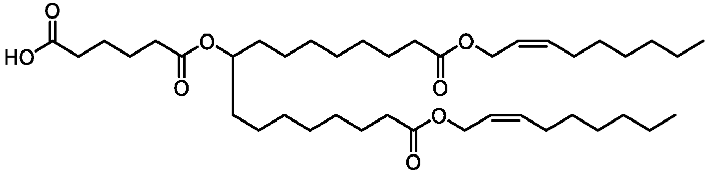 |
| 98 | 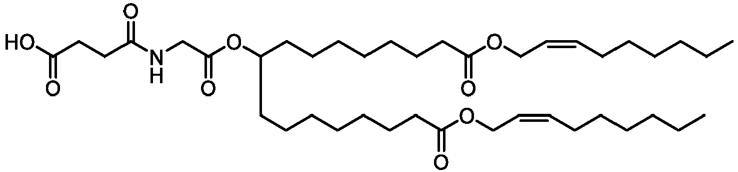 |
| 99 | 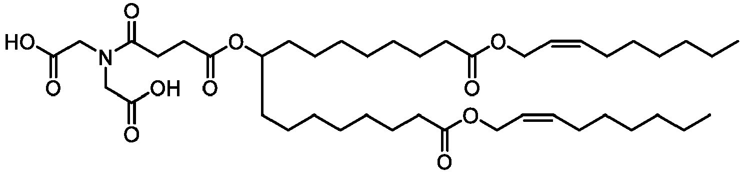 |
| 100 | 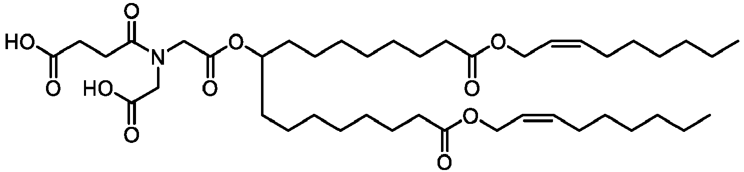 |
| 101 | 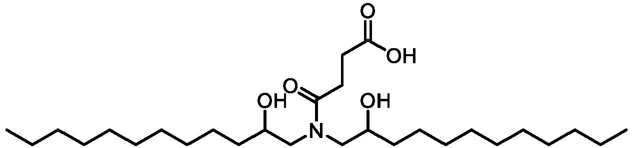 |
| 102 | 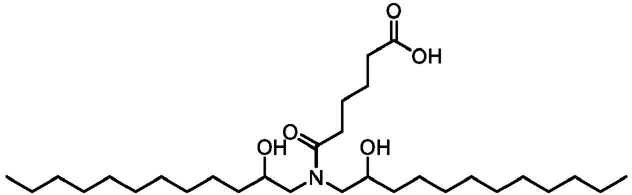 |
| 103 | 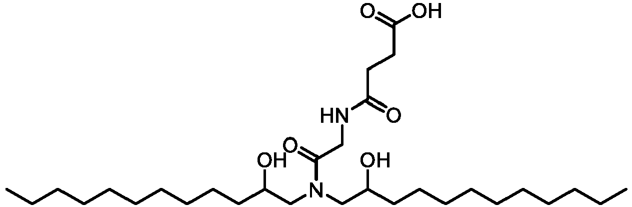 |
| 104 | 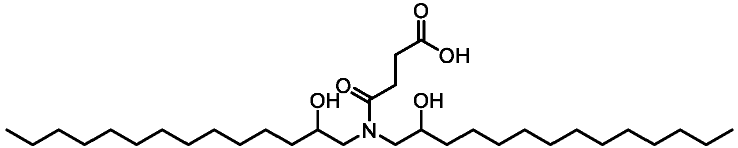 |
| 105 | 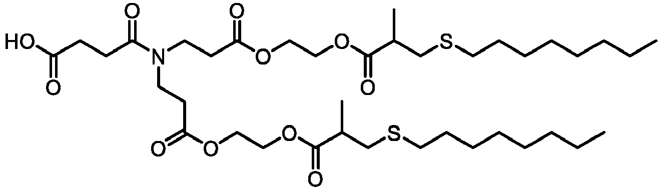 |
| 106 | 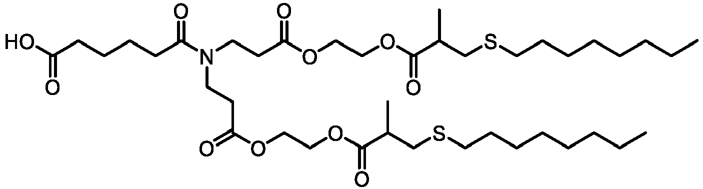 |
| 107 | 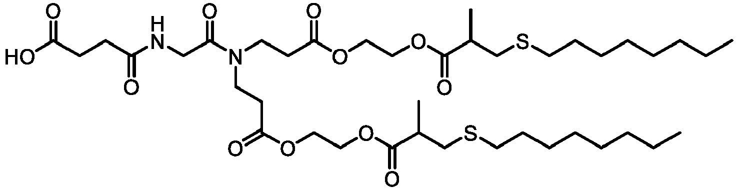 |
| 108 | 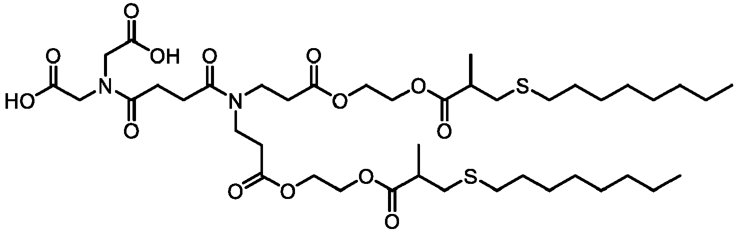 |
| 109 | 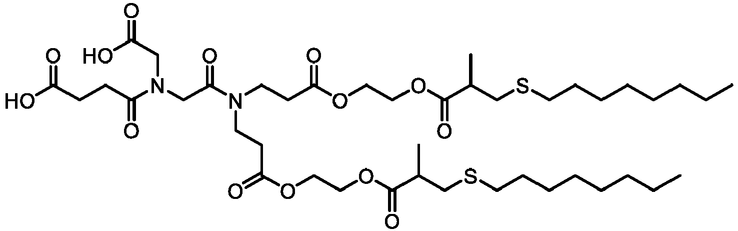 |
| 110 | 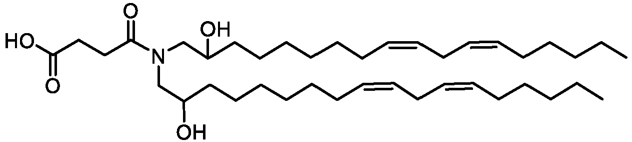 |
| 111 | 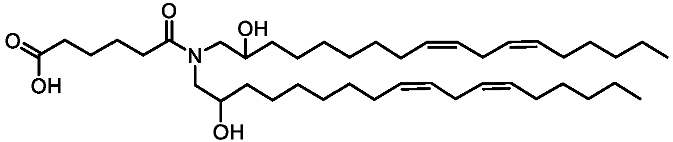 |
| 112 | 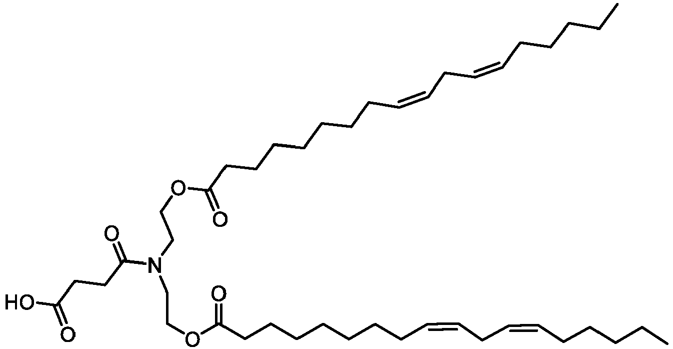 |
| 113 | 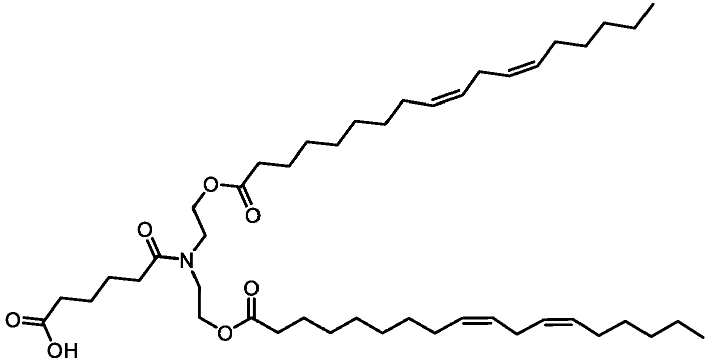 |
| 114 | 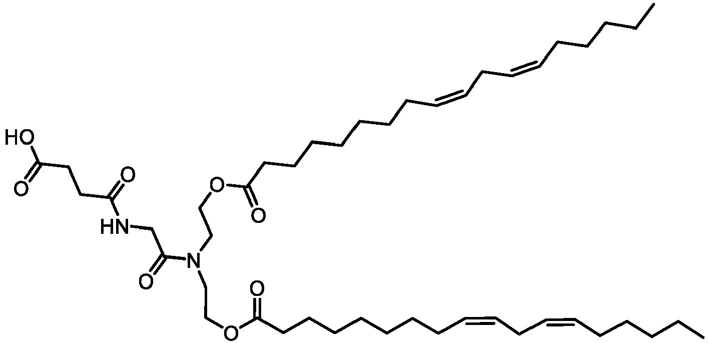 |
| 115 | 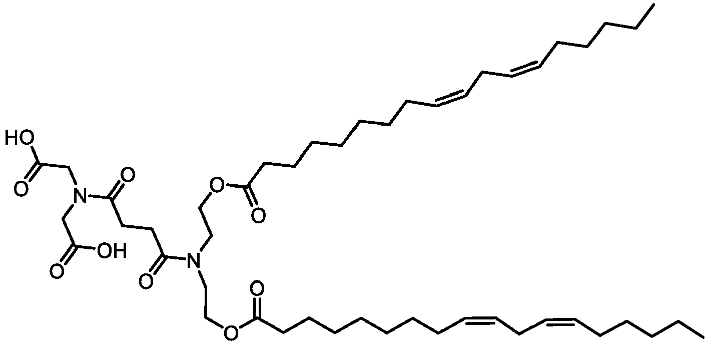 |
| 116 | 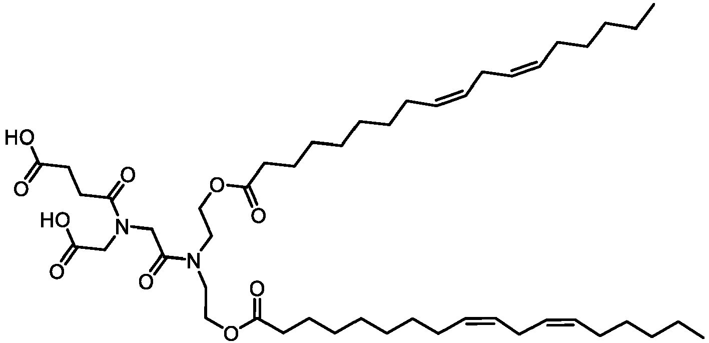 |
| 117 | 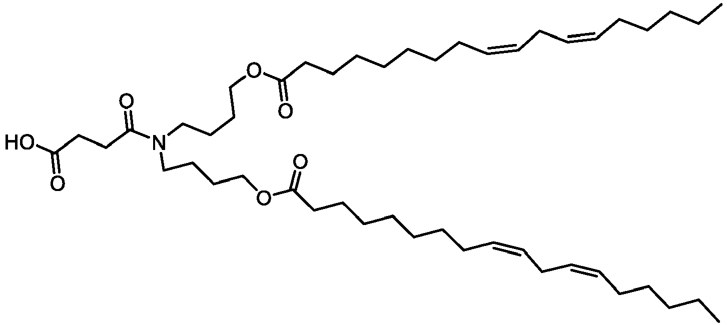 |
| 118 | 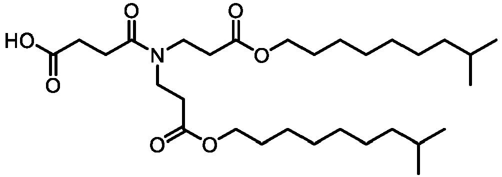 |
| 119 | 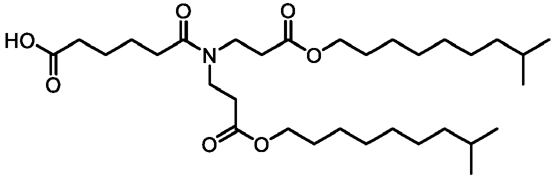 |
| 120 | 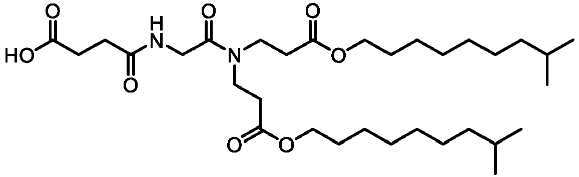 |
| 121 | 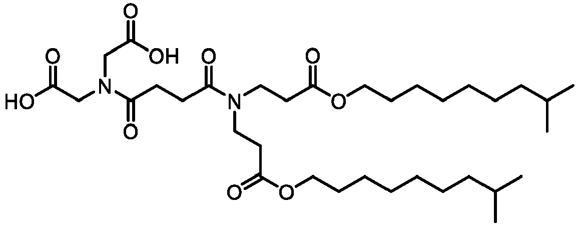 |
| 122 | 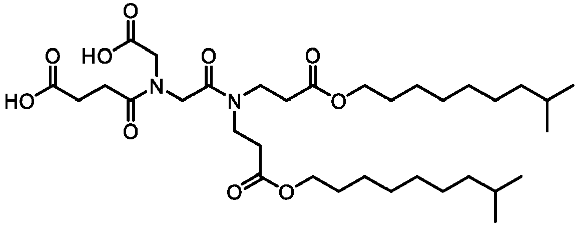 |
| 123 | 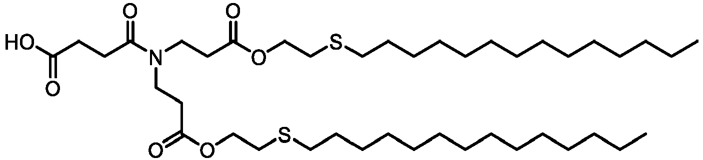 |
| 124 | 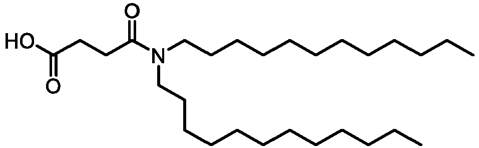 |
| 125 | 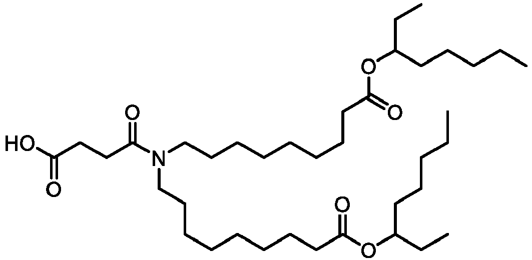 |
| 126 | 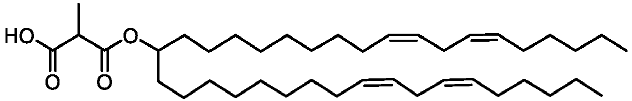 |
| 127 | 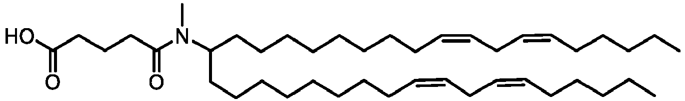 |
| 128 | 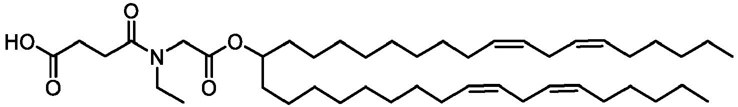 |
| 129 | 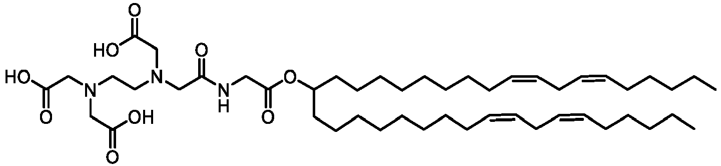 |
| 130 | 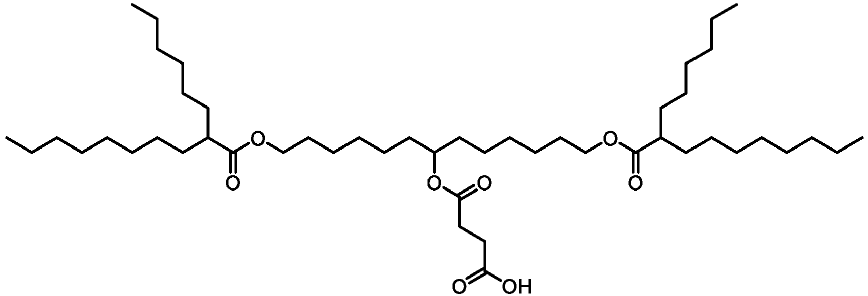 |
| 131 | 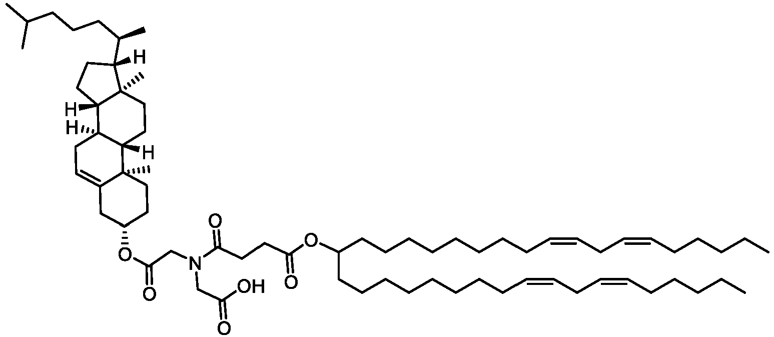 |
| 132 | 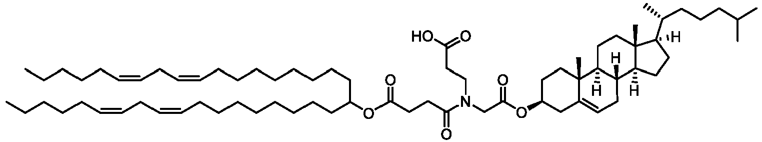 |
| 133 | 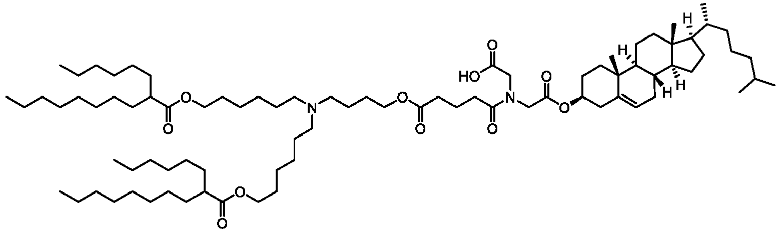 |
| 134 | 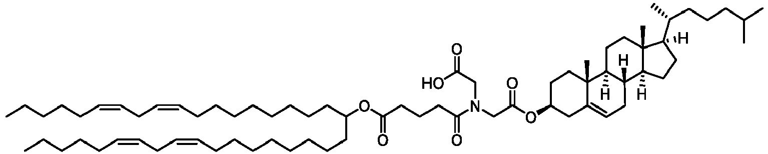 |
| 135 | 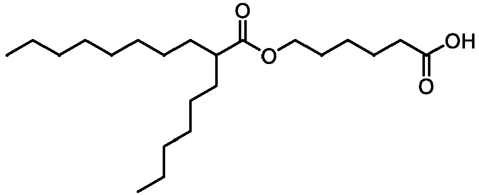 |
| 136 | 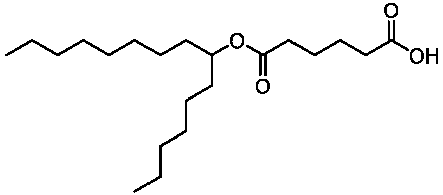 |
| 137 | 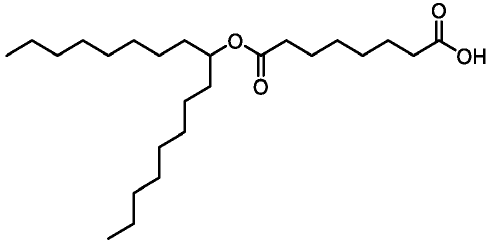 |

The present disclosure further provides a lipid nanoparticle which can target different tissues and organs for drug delivery. The lipid nanoparticle comprises a cationic lipid, the lipid compound provided in this disclosure or a pharmaceutically acceptable salt, a prodrug, a stereoisomer thereof described herein, wherein the cationic lipid is selected from one of or a combination of more than one of a permanently cationic lipid and/or an ionizable cationic lipid; the lipid compound is a compound from formula I, preferably, the lipid compound is selected from compounds as shown in Table 1 or a combination thereof. Furthermore, the lipid nanoparticle may further comprise at least one helper lipid, which is mixed with a drug to achieve encapsulation and specific delivery to a specific tissue and organ. The specificity for the tissue and the organ is achieved by the lipid compound or the pharmaceutically acceptable salt, the prodrug, or the stereoisomer thereof in the lipid nanoparticle, preferably by a combination of the lipid compound or the pharmaceutically acceptable salt, the prodrug, or the stereoisomer thereof and the cationic lipid, and further preferably by a combination of the lipid compound or the pharmaceutically acceptable salt, the prodrug, or the stereoisomer thereof, the cationic lipid, and the helper lipid. In some aspects of the present disclosure, provided is a lipid nanoparticle comprising:
(1) a lipid compound or a pharmaceutically acceptable salt, a prodrug, a stereoisomer, or a deuteride thereof;
(2) a cationic lipid; and
(3) a helper lipid.

The lipid nanoparticle preferentially delivers the therapeutic/prophylactic agent to the following target organs: lung, heart, brain, spleen, lymph node, bone, skeletal muscle, stomach, small intestine, large intestine/colon and rectum, kidney, bladder, breast, testis, ovary, uterus, thymus, brainstem, cerebellum, cerebrum, spinal cord, eye, ear, tongue, or skin. Preferably, the target organ is the spleen.

The cationic lipid in the lipid nanoparticle comprises an ammonium group that is positively charged at a given pH and comprises at least two hydrophobic groups. The cationic lipid is a dendrimer or dendron. The cationic lipid comprises at least two C6-C24 hydrocarbyl groups. The cationic lipid may be one of or a combination of more than one of a permanently cationic lipid and/or an ionizable cationic lipid. The permanently cationic lipid may be selected from one or more of DOTAP, DODMA, DSTAP, DMTAP, DDA, and DOBAQ; the ionizable cationic lipid may be selected from one of or a combination of more than one of SM-102, Lipid 5, A6, DC-chol, C12-200, CKK-E12, 5A2-SC8, G0-C14, OF-2, 306Oi10, OF-Deg-Lin, 92-0175, OF-C4-Deg-Lin, A18-iso5-2DC18, TT3, FTT5, BAMEA-O16B, Vc-Lipid, C14-4, Lipid 14, 4A3-Cit, and ssPalmO-Phe.

In the lipid nanoparticle described above, the cationic lipid and the lipid compound or the pharmaceutically acceptable salt, the prodrug, or the stereoisomer thereof are in a molar ratio of 1:1 to 11:1.

Further, the lipid nanoparticle may comprise a helper lipid. The helper lipid is optionally one or more of a phospholipid, a steroid, a polymer conjugated lipid, and a modifiable lipid.

Preferably, the phospholipid is selected from any one of DOPE, DSPC, DPPC, DMPC, DOPC, POPC and SM or a combination thereof.

Preferably, the steroid is selected from one or more of cholesterol, sitosterol, stigmasterol, and ergosterol.

Preferably, the polymer in the polymer conjugated lipid is a high-molecular-weight compound formed by covalent bonding of one or more small-molecule repeating units; the polymer may be selected from polyethylene glycol, polylactic acid, polyamide, cationic polymer, poly sarcosine (pSar), poly lactic-co-glycolic acid (PLGA), polyamino acid, polypeptide, polypeptoid, etc.; preferably, the polymer conjugated lipid is selected from a polyethylene glycol conjugated lipid; further, the polyethylene glycol conjugated lipid is selected from one or more of PEG1000-DMG, PEG5000-DMG, PEG2000-DMG, and PEG2000-DSPE.

Preferably, the modifiable lipid includes lipids modified by small-molecule compounds, vitamins, carbohydrates, peptides, proteins, nucleic acids, lipopolysaccharides, inorganic molecules or particles, metal ions or particles, and combinations of the substances described above.

In the lipid nanoparticle, the cationic lipid, the lipid compound or the pharmaceutically acceptable salt, the prodrug, or the stereoisomer thereof, and the helper lipid are in a molar ratio of 1:(0.1-1):(0.5-2).

In other aspects, the present invention provides a composition comprising a therapeutic or prophylactic agent and the lipid nanoparticle described above.

In a preferred instance, the therapeutic/prophylactic agent is a nucleic acid.

Preferably, the nucleic acid includes any and all forms of nucleic acid molecules, including but not limited to, single-stranded DNA, double-stranded DNA, single-stranded RNA, double-stranded RNA, short isomers, plasmid DNA, complementary DNA/cDNA, antisense oligonucleotide/ASO, small interfering nucleic acid/siRNA, small activating nucleic acid/saRNA, asymmetric interfering nucleic acid/aiRNA, micro nucleic acid/miRNA, miRNA inhibitors (agomir, antagomir), Dicer enzyme substrate nucleic acid, small hairpin nucleic acid/shRNA, transfer RNA (tRNA), messenger RNA/mRNA, circular RNA/circRNA, self-amplifying mRNA/samRNA, aptamers, and other forms of nucleic acid molecules known in the art.

The above nucleic acid molecules may include natural nucleotides, or may include nucleotide mimics or functional analogs, or may include chemically modified forms of nucleotides.

Functional nucleotide analogs include, but are not limited to, one of or a combination of more than one of a locked nucleic acid (LNA), a peptide nucleic acid (PNA), and a morpholine ring oligonucleotide nucleic acid mimic or functional analog.

The chemical modification of nucleotides may be located on a backbone bond of the nucleic acid molecule. The backbone bond may be modified by the replacement of one or more oxygen atoms. The modification to the backbone bond may include replacing at least one phosphodiester bond with a phosphorothioate bond.

The chemical modification of nucleotides may be located on a nucleoside. The modification on the nucleoside may be located on the sugar and base of the nucleoside. The sugar on the nucleoside may be selected from one or more of: 2'-fluororibose, ribose, 2'-deoxyribose, arabinose, and hexose.

The chemically modified forms of nucleotides may be selected from one or more of: 5-methylcytosine, pseudouridine, 1-methylpseudouridine, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthio-adenine, 2-methoxy-adenine, inosine, 1-methylinosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

In a preferred example, the nucleic acid is an mRNA capable of encoding at least one antigen or a fragment thereof or an epitope thereof, or encoding a certain therapeutic protein, the antigen being selected from a pathogenic antigen, such as a tumor-associated antigen or a pathogenic microbial antigen. The mRNA may be a monocistronic mRNA or a polycistronic mRNA.

The present disclosure further provides use of the above composition in the preparation of medicines.

The present disclosure further provides a drug comprising the composition described above and a pharmaceutically acceptable auxiliary material. The pharmaceutical auxiliary materials refer to excipients and additives and ajuvents used in drug production and prescription dispensing and are substances other than the active ingredient, which have been rationally evaluated in safety and are contained in a pharmaceutical formulation. In addition to shaping, serving as vehicles and enhancing stability, pharmaceutical auxiliary materials also have such important functions as solubilization, aiding dissolution, sustaining or controlling release, etc. They are important ingredients which possibly affect the quality, safety and effectiveness of drugs. According to the effect and the purpose, pharmaceutical auxiliary materials may be classified into solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesives, integrating agents, permeation promotors, pH regulators, buffers, plasticizers, surfactants, foaming agents, defoaming agents, thickeners, inclusion agents, humectants, absorbents, diluents, flocculants, deflocculants, filter aids, release retardants, etc.

The benefits of the present disclosure are as follows:
The present disclosure provides a novel lipid compound or a pharmaceutically acceptable salt, a prodrug, or a stereoisomer thereof, the lipid compound and derivatives thereof being capable of selectively delivering a therapeutic/prophylactic agent to a target organ.

The present disclosure further provides a novel lipid nanoparticle, which can deliver a nucleic acid component to a specific organ, particularly to organs other than the liver preferentially, so as to provide more options for the delivery of nucleic acid drugs, gene vaccines and so on, and particularly have important significance for the development and application of nucleic acid prophylactic and therapeutic agents.

Traditional lipid nanoparticles generally deliver to the liver in a targeted way. According to the present disclosure, by adding the lipid compound or the pharmaceutically acceptable salt, the prodrug, or the stereoisomer thereof to the lipid nanoparticle,t the targeting property for organs is greatly increased. In the lipid nanoparticle such as TMF8 and TMF100-TMF109 to which the lipid compound of the present disclosure is added, the ratio of the distribution (mean fluorescence intensity) of nucleic acid drugs in the spleen to that in the liver is 7 to 99 (Table 5). However, in traditional lipid nanoparticles without the addition of the lipid compound of the present disclosure, there is a higher delivery efficiency in the liver (Ansell, S. M.; Du, X. Novel Lipids and Lipid Nanoparticle Formulations for Delivery of Nucleic Acids. WO2017075531 A1).

Therefore, the lipid compound of the present disclosure and the lipid nanoparticle comprising same play an important role in the targeting property for delivered organs.

### BRIEF DESCRIPTION OF THE DRAWINGS

FIG. 1 shows a nuclear magnetic resonance spectrum of compound 40.
FIG. 2 shows a nuclear magnetic resonance spectrum of compound 44.
FIG. 3 shows a nuclear magnetic resonance spectrum of compound 48.
FIG. 4 shows a nuclear magnetic resonance spectrum of compound 70.
FIG. 5 shows a nuclear magnetic resonance spectrum of compound 130.
FIG. 6 shows a nuclear magnetic resonance spectrum of compound 131.
FIG. 7 shows a nuclear magnetic resonance spectrum of compound 132.
FIG. 8 shows a nuclear magnetic resonance spectrum of compound 133.
FIG. 9 shows a nuclear magnetic resonance spectrum of compound 134.
FIG. 10 shows a nuclear magnetic resonance spectrum of compound 135.
FIG. 11 shows a nuclear magnetic resonance spectrum of compound 136.
FIG. 12 shows a nuclear magnetic resonance spectrum of compound 137.
FIG. 13 is a graph showing fluorescence signals of different organs in mice 4 h after administration by intravenous injection of a sample of lipid nanoparticle TMF107 loaded with Luciferase mRNA. As shown in the graph, the fluorescence signal is strongest in the spleen of the mice, and very weak in the liver. It is indicated that the expression of Luciferase mRNA in the spleen is higher than that in the liver.
FIG. 14 shows summary data of fluorescence intensity ratios for different lipid nanoparticles delivering luciferase mRNA in the spleen and liver.

### DETAILED DESCRIPTION

### A. Chemical and Formulation Definitions

When used in the context of a chemical group, "hydrogen" refers to -H; "deuterium" refers to ²H or D; "hydroxyl" refers to -OH; "oxo" refers to =O; "carbonyl" refers to -C(=O)-; "carboxyl" refers to -C(=O)OH (also written as -COOH or -CO₂H); "halo" independently refers to -F, -Cl, -Br or -I; "amino" refers to -NH₂; "hydroxyamino" refers to -NHOH; "nitro" refers to -NO₂; imino refers to =NH; "cyano" refers to -CN; "isocyanate" refers to -N=C=O; "azido" refers to -N₃; in the context of a monovalent group, "phosphate" refers to -OP(O)(OH)₂ or a deprotonated form thereof; in the context of a divalent group, "phosphate" refers to -OP(O)(OH)O- or a deprotonated form thereof; "sulfydryl" refers to -SH; and "thio" refers to =S; "sulfonyl" refers to -S(O)₂-; "hydroxysulfonyl" refers to -S(O)₂OH; "sulfonamide" refers to -S(O)₂NH₂; and "sulfinyl" refers to -S(O)-. In the context of a chemical formula, the symbol "-" refers to a single bond, "=" refers to a double bond, and "≡" refers to a triple bond. The symbol "----" represents an optional bond, which is a single or double bond, if present. When drawn perpendicularly across a bond, the symbol " 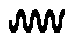 " indicates the point of attachment of the group. It should be noted that the point of attachment is generally identified only for larger groups in this manner to aid the reader in unequivocally identifying the point of attachment. The symbol " 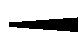 " refers to a single bond, wherein the group attached to the butt end of the wedge "emerges from the paper". The symbol " 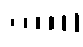 " refers to a single bond, wherein the group attached to the butt end of the wedge "goes into the paper". The symbol " 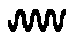 " refers to a single bond, wherein the geometry (e.g., E or Z) around the double bond is undefined. Thus, both options and combinations thereof are contemplated. Any undefined valence on an atom of a structure shown in the present application implicitly represents a hydrogen atom bonded to the atom. Bold dots on a carbon atom indicate that the hydrogen attached to the carbon is oriented out of the plane of the paper. The term "alkyl" as used without the modifier "substituted" represents a monovalent saturated aliphatic group having a carbon atom as the point of attachment, having a linear or branched acyclic structure, and no atoms other than carbon and hydrogen. The groups -CH₃(Me), -CH₂CH₃(Et), -CH₂CH₂CH₃(n-Pr or propyl), - CH(CH₃)₂(i-Pr, iPr or isopropyl), -CH₂CH₂CH₂CH₃(n-Bu), -CH(CH₃)CH₂CH₃(sec-butyl), - CH₂CH(CH₃)₂(isobutyl), -C(CH₃)₃(*tert*-butyl, t-Bu or tBu) and -CH₂C(CH₃)₃(neopentyl) are non-limiting examples of alkyl. The term "dialkyl" as used without the modifier "substituted" represents a divalent saturated aliphatic group having 1 or 2 saturated carbon atoms as the point of attachment, having a linear or branched acyclic structure, no carbon-carbon double or triple bonds, and no atoms other than carbon and hydrogen. The groups -CH₂-(methylene), -CH₂CH₂-, -CH₂C(CH₃)₂CH₂- and -CH₂CH₂CH₂- are non-limiting examples of dialkyl. "Alkane" represents the class of compounds having formula H-R, wherein R is alkyl, and the term is as defined above. When any of these terms is used together with the modifier "substituted", one or more hydrogen atoms have been independently replaced by -OH, -F, -Cl, -Br, -I, -NH₂, -NOz, -COzH, -CO₂CH₃, -CN, -SH, - OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, - OC(O)CH₃, -NHC(O)CH₃, -S(O)zOH or -S(O)₂NH₂. The following groups are non-limiting examples of substituted alkyl: -CH_{z}OH, -CH₂Cl, -CF₃, -CH₂CN, -CH₂C(O)OH, -CH₂C(O)OCH₃, -CH₂C(O)NH₂, - CH₂C(O)CH₃, -CH2OCH₃, -CH₂OC(O)CH₃, -CH₂NH₂, -CH₂N(CH₃)₂, and -CH₂CH₂Cl. The term "haloalkyl" is a subset of substituted alkyl, wherein hydrogen atom substitution is limited to halo (i.e., -F, -Cl, -Br, or -I), such that no atoms other than carbon, hydrogen and halogen are present. The group -CH₂Cl is one non-limiting example of haloalkyl. The term "fluoroalkyl" is a subset of substituted alkyl, wherein hydrogen atom substitution is limited to fluoro, such that no atoms other than carbon, hydrogen and fluorine are present. The groups -CH₂F, -CF₃ and -CH₂CF₃ are non-limiting examples of fluoroalkyl.

The term "alkenyl" as used without the modifier "substituted" represents a monovalent unsaturated aliphatic group having a carbon atom as the point of attachment, having a linear or branched acyclic structure, at least one non-aromatic carbon-carbon double bond, no carbon-carbon triple bonds, and no atoms other than carbon and hydrogen. Non-limiting examples include: -CH=CH₂(vinyl), -CH=CHCH₃, -CH=CHCH₂CH₃, - CH₂CH=CH₂(allyl), -CH₂CH=CHCH₃, and -CH=CHCH=CH₂. The term "alkenediyl" as used without the modifier "substituted" represents a divalent unsaturated aliphatic group having 2 carbon atoms as the point of attachment, having a linear or branched, linear or branched acyclic structure, at least one non-aromatic carbon-carbon double bond, no carbon-carbon triple bonds, and no atoms other than carbon and hydrogen. The groups -CH=CH-, -CH=C(CH₃)CH₂-, -CH=CHCH₂- and -CH₂CH=CHCH₂- are non-limiting examples of alkenediyl groups. It should be noted that although the alkenediyl group is aliphatic, once attached at both ends, it is not excluded that the group forms part of an aromatic structure. The terms "olefin" and "chain olefin" are synonymous and represent the class of compounds having formula H-R, wherein R is alkenyl, and the terms are as defined above. Similarly, the terms "terminal olefin" and "α-olefin" are synonymous and represent olefin having exactly one carbon-carbon double bond, wherein the bond is part of vinyl at the terminus of the molecule. When any of these terms is used together with the modifier "substituted", one or more hydrogen atoms have been independently replaced by -OH, -F, -Cl, -Br, -I, -NH₂, -NOz, -CO₂H, -CO₂CH₃, -CN, -SH, -OCH₃, - OCH₂CH₃, -C(O)CH₃, -NHCH₃, -NHCH₂CH₃, -N(CH₃)₂, -C(O)NH₂, -C(O)NHCH₃, -C(O)N(CH₃)₂, - OC(O)CH₃, -NHC(O)CH₃, -S(O)zOH or -S(O)₂NH₂. The groups -CH=CHF, -CH=CHCl and -CH=CHBr are non-limiting examples of substituted alkenyl.

The term "alkynyl" as used without the modifier "substituted" represents a monovalent unsaturated aliphatic group having a carbon atom as the point of attachment, having a linear or branched acyclic structure, at least one carbon-carbon triple bond, and no atoms other than carbon and hydrogen. The term alkynyl as used herein does not preclude the presence of one or more non-aromatic carbon-carbon double bonds. The groups -C≡CH, - C≡CCH₃ and -CH₂C≡CCH₃ are non-limiting examples of alkynyl. "Alkyne" represents the class of compounds having formula H-R, wherein R is alkynyl. When any of these terms is used together with the modifier "substituted", one or more hydrogen atoms have been independently replaced by -OH, -F, -Cl, -Br, -I, -NH₂, - NO₂, -CO₂H, -CO₂CH₃, -CN, -SH, -OCH₃, -OCH₂CH₃, -C(O)CH₃, -NHCH₃, -NHCH₂CH₃, -N(CH₃)₂, - C(O)NH₂, -C(O)NHCH₃, -C(O)N(CH₃)₂, -OC(O)CH₃, -NHC(O)CH₃, -S(O)zOH or -S(O)₂NH₂.

"Prodrug" refers to a compound, such as a therapeutic agent that can be converted into a biologically active compound under physiological conditions or by dissolution. The prodrug is generally rapidly converted *in vivo* to yield a parent compound, for example, by hydrolysis in blood. The prodrug compound generally has the advantage of solubility, histocompatibility, or delayed release in mammalian organisms. The term "prodrug" is also meant to include any covalently bonded carriers that release active compounds *in vivo* when the prodrug is administered to a mammalian subject. The prodrug includes a compound where hydroxyl, amino or sulfhydryl is bonded to any group that, when the prodrug is administered to the mammalian subject, cleaves to form free hydroxyl, free amino, or free sulfhydryl, respectively. Examples of the prodrug include, but are not limited to, acetate, formate and benzoate derivatives or amide derivatives of amine functional groups and the like.

"Pharmaceutically acceptable salt" refers to a salt of a compound of the present disclosure that is pharmaceutically acceptable as defined above and that has a desired pharmacological activity. Such salts include acid addition salts formed with the following acids: inorganic acids such as hydrochloric acid, hydrobromic acid, sulfuric acid, nitric acid, phosphoric acid, and the like; or organic acids such as 1,2-ethanedisulfonic acid, 2-hydroxyethanesulfonic acid, 2-naphthalenesulfonic acid, 3-phenylpropionic acid, 4,4'-methylene-bis(3-hydroxy-2-ene-1-methanoic acid), 4-methylbicyclo[2.2.2]oct-2-ene-1-methanoic acid, acetic acid, aliphatic monocarboxylic and dicarboxylic acids, aliphatic sulfuric acid, aromatic sulfuric acid, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, lauryl sulfuric acid, maleic acid, malic acid, malonic acid, mandelic acid, methanesulfonic acid, muconic acid, o-(4-hydroxybenzoyl)benzoic acid, oxalic acid, *p-*chlorobenzenesulfonic acid, phenyl-substituted alkanoic acid, propionic acid, p-toluenesulfonic acid, pyruvic acid, salicylic acid, stearic acid, succinic acid, tartaric acid, *tert*-butylacetic acid, trimethylacetic acid, and the like. The pharmaceutically acceptable salts further include base addition salts which may be formed when an acidic proton present is capable of reacting with an inorganic or organic base. The acceptable inorganic bases include sodium hydroxide, sodium carbonate, potassium hydroxide, aluminum hydroxide, and calcium hydroxide. The acceptable organic bases include ethanolamine, diethanolamine, triethanolamine, tromethamine, N-methylglucainine and the like. It should be recognized that the particular anion or cation forming part of any salt of the present disclosure is not critical so long as the salts as a whole are pharmacologically acceptable.

"Stereoisomers" or "optical isomers" are isomers of such a given compound, wherein the same atom is bonded to the same other atom; however, the three-dimensional configuration of those atoms is different. "Enantiomers" are stereoisomers of a given compound that are mirror images of each other as left and right hands. "Diastereoisomers" are stereoisomers of a given compound that are not enantiomers. Chiral molecules contain a chiral center (also referred to as a stereocenter or a stereogenic center), which is any point (although not necessarily an atom) in the molecule that carries multiple groups, such that the interchange of any 2 groups produces a stereoisomer. In organic compounds, the chiral center is typically a carbon, phosphorus, or sulfur atom, although other atoms may also be stereocenters in organic and inorganic compounds. A molecule may have multiple stereocenters, giving rise to many of its stereoisomers. In compounds whose stereoisomerism is due to tetrahedral stereogenic centers (e.g., tetrahedral carbon), it is assumed that the total number of possible stereoisomers does not exceed 2n, wherein n is the number of tetrahedral stereogenic centers. Molecules with symmetry often have a number that is smaller than the maximum possible number of stereoisomers. A 50:50 mixture of enantiomers is referred to as a racemic mixture. Alternatively, a mixture of enantiomers may be enantiomerically enriched such that one enantiomer is present in an amount greater than 50%. Generally, enantiomers and/or diastereomers may be resolved or separated using techniques known in the art. It is contemplated that for any stereogenic center or chiral axis for which stereochemistry has not been defined, the stereogenic center or chiral axis may exist in its R form, S form, or as a mixture of the R form and the S form (including racemic and non-racemic mixtures). As used herein, the phrase "substantially free of other stereoisomers" means that the composition contains 15% or less, more preferably 10% or less, even more preferably 5% or less, or most preferably 1% or less of another or more stereoisomers.

Deuterium (²H or D) is a stable and nonradioactive isotope of hydrogen, which has approximately twice the mass of hydrogen (H), the most common isotope of hydrogen.

"Prevention" or "preventing" includes: (1) inhibiting the onset of a disease in a subject or patient who may be at risk of and/or susceptible to the disease, but who has not yet experienced or exhibited any or all of the conditions or symptoms of the disease; and/or (2) slowing the onset of the conditions or symptoms of a disease in a subject or patient who may be at risk of and/or susceptible to the disease, but who has not experienced or exhibited any or all of the conditions or symptoms of the disease.

"Treatment" or "treating" includes: (1) inhibiting a disease (e.g., arresting the further development of the conditions and/or symptoms) in a subject or patient experiencing or exhibiting the conditions or symptoms of the disease, (2) ameliorating a disease (e.g., reversing the conditions and/or symptoms) in a subject or patient experiencing or exhibiting the conditions or symptoms of the disease, and/or (3) effecting any measurable mitigation of a disease in a subject or patient experiencing or exhibiting the conditions or symptoms of the disease.

"Protein", "polypeptide" or "peptide" refers to a polymer of amino acid residues, including a wide range of protein molecules such as cytokines, chemokines, interleukins, interferons, growth factors, coagulation factors, anticoagulants, blood factors, bone morphogenic proteins, immunoglobulins, and enzymes. Some non-limiting examples of therapeutic proteins include the following therapeutic proteins, or fragments, variants or derivatives thereof: therapeutic proteins for the treatment of metabolic or endocrine disorders, comprising acid sphingomyelinase, adipotide, agalsidase-β, alglucosidase, α-galactosidase A, α-glucosidase, α-L-iduronidase, α-N-acetylglucosidase, amphiregulin, angiogenin (Ang1, Ang2, Ang3, Ang4, ANGPTL2, ANGPTL3, ANGPTL4, ANGPTL5, ANGPTL6, ANGPTL7), β-cellulin, β-glucuronidase, bone morphogenetic proteins BMPs (BMP1, BMP2, BMP3, BMP4, BMP5, BMP6, BMP7, BMP8a, BMP8b, BMP10, BMP15), CLN6 protein, epidermal growth factor (EGF), epigen, epiregulin, fibroblast growth factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, FGF-23), galsulphase, ghrelin, glucocerebrosidase, GM-CSF, heparin-binding EGF-like growth factor (HB-EGF), hepatocyte growth factor HGF, hepcidin, human albumin, increased albumin loss, idursulfase (iduronate-2-sulfatase), integrins αVβ3, αVβ5 and α5β1, iduronate sulfatase, laronidase, N-acetylgalactosamine-4-sulfatase (rhASB; galsulase, arylsulfatase A (ARSA), arylsulfatase B (ARSB)), N-acetylglucosamine-6-sulfatase, nerve growth factor (NGF, brain-derived neurotrophic factor (BDNF), neurotrophin-3 (NT-3), and neurotrophin-4/5(NT-4/5), neuregulin (NRG1, NRG2, NRG3, NRG4), neuropilin (NRP-1, NRP-2), myostatin, platelet-derived growth factor (PDGF (PDFF-A, PDGF-B, PDGF-C, PDGF-D), TGF β receptor (endothelial factor, TGF-β1 receptor, TGF-β2 receptor, TGF-β3 receptor), thrombopoietin (THPO) (megakaryocyte growth and development factor (MGDF)), transforming growth factor (TGF(TGF-a, TGF-β (TGFβ1, TGFβ2, and TGFβ3))), VEGF (VEGF-A, VEGF-B, VEGF-C, VEGF-D, VEGF-E, VEGF-F, and PIGF), nesiritide, trypsin, adrenocorticotropic hormone (ACTH), atrial natriuretic peptide (ANP), cholecystokinin, gastrin, leptin, oxytocin, somatostatin, vasopressin (antidiuretic hormone), calcitonin, exenatide, growth hormone (GH), auxin, insulin, insulin-like growth factor 1IGF-1, mecasermin rinfabate, IGF-1 analogs, mecasermin, IGF-1 analogs, pegvisomant, pramlintide, teriparatide (human parathyroid hormone residue 1-34), becaplermin, dibotermin-α (bone morphogenetic protein 2), histrelin acetate (gonadotropin releasing hormone; GnRH), octreotide, and palifermin (keratinocyte growth factor; KGF); therapeutic proteins for the treatment of hematologic disorder, circulatory disease, respiratory disease, cancer or neoplastic disease, infectious disease or immunodeficiency, comprising alteplase (tissue plasminogen activator; tPA), anistreplase, antithrombin III (AT-III), bivalirudin, darbepoetin-α, drotrecogin-α (activated protein C, erythropoietin, epoetin-α, hemoglobin-promoting auxin, erythropoietin, factor IX, factor VIIa, factor VIII, lepirudin, protein C concentrate, reteplase (deletion mutant protein of tPA), streptokinase, tenecteplase, urokinase, angiostatin, anti-CD22 immunotoxin, dinleukin-toxin linker, immunocyanin, MPS (metallopanstimulin), aflibercept, endostatin, collagenase, human deoxyribonuclease I, streptodornase, hyaluronidase, papain, L-asparaginase, Peg-asparaginase, rasburicase, human chronic gonadotropin (HCG), human follicle stimulating hormone (FSH), luteinizing hormone-α, prolactin, α-1-protease inhibitors, lactase, pancreatin (lipase, amylase, protease), adenosine deaminase (pegademase bovine, PEG-ADA), abatacept, alefacept, anakinra, etanercept, interleukin-1 (IL-1) receptor antagonist, thymosin, TNF-α antagonist, enfuvirtide, and thymosin α1; therapeutic proteins selected from adjuvants or immunostimulatory proteins, comprising: human adjuvant proteins, in particular the pattern recognition receptors TLR1, TLR2, TLR3, TLR4, TLR5, TLR6, TLR7, TLR8, TLR9, TLR10, TLR11; NOD1, NOD2, NOD3, NOD4, NODS, NALP1, NALP2, NALP3, NALP4, NALP5, NALP6, NALP7, NALP8, NALP9, NALP10, NALP11, NALP12, NALP13, NALP14, 1IPAF, NAIP, CIITA, RIG-I, MDA5, and LGP2, signal transducers for TLR signaling, comprising adaptor proteins, including, for example, Trif and Cardif; small-GTPase signaling components (RhoA, Ras, Rac1, Cdc42, Rab, etc.), PIP signaling components (PI3K, Src-kinase, etc.), MyD88-dependent signaling components (MyD88, IRAK1, IRAK2, IRAK4, TIRAP, TRAF6, etc.), MyD88-independent signaling components (TICAM1, TICAM2, TRAF6, TBK1, IRF3, TAK1, IRAK1, etc.); activated kinases, including, for example, Akt, MEKK1, MKK1, MKK3, MKK4, MKK6, MKK7, ERK1, ERK2, GSK3, PKC kinase, PKD kinase, GSK3 kinase, JNK, p38MAPK, TAK1, IKK, and TAK1; activated transcription factors, including, for example, NF-κB, c-Fos, c-Jun, c-Myc, CREB, AP-1, Elk-1, ATF2, IRF-3, IRF-7, heat shock proteins such as HSP10, HSP60, HSP65, HSP70, HSP75 and HSP90, gp96, fibrinogen, the TypIII repeat addition domain A of fibronectin; or components of the complement system, including C1q, MBL, C1r, C1s, C2b, Bb, D, MASP-1, MASP-2, C4b, C3b, C5a, C3a, C4a, C5b, C6, C7, C8, C9, CR1, CR2, CR3, CR4, C1qR, C1INH, C4bp, MCP, DAF, H, I, P, and CD59, or induced target genes including, for example, β-defensin, cell surface protein; or human adjuvant proteins, including trif, flt-3 ligand, Gp96 or fibronectin, cytokines that induce or enhance the innate immune response, including IL-1α, IL1β, IL-2, IL-6, IL-7, IL-8, IL-9, IL-12, IL-13, IL-15, IL-16, IL-17, IL-18, IL-21, IL-23, TNF-α, IFN-α, IFN-β, IFN-γ, GM-CSF, G-CSF, M-CSF; chemokines, including IL-8, IP-10, MCP-1, MIP-1α, RANTES, eotaxin, CCL21; cytokines released by macrophages, including IL-1, IL-6, IL-8, IL-12 and TNF-α; and IL-1R1 and IL-1α; bacterial (adjuvant) proteins, in particular bacterial heat shock proteins or chaperones, including Hsp60, Hsp70, Hsp90, Hsp100; OmpA (outer membrane protein) from gram-negative bacteria; bacterial porins, including OmpF; bacterial toxins, including pertussis toxin (PT) from Bordetella pertussis, pertussis adenylate cyclase toxins CyaA and CyaC from Bordetella pertussis, PT-9K/129G mutant from pertussis toxin, tetanus toxin, cholera toxin (CT), cholera toxin B-subunit, CTK63 mutant from cholera toxin, CTE112K mutant from CT, Escherichia coli heat-labile enterotoxin (LT), B subunit from heat-labile enterotoxin (LTB), Escherichia coli heat-labile enterotoxin mutants with reduced toxicity, including LTK63 and LTR72; phenol soluble modulins; neutrophil activating protein (HP-NAP) from Helicobacter pylori; surfactant protein D; outer surface protein A lipoproteins from Borrelia burgdorferi, and Ag38(38kDa antigen) from Mycobacterium tuberculosis; proteins from bacterial pili; enterotoxin CT of Vibrio cholerae, pilin from pili of gram-negative bacteria, and surfactant protein A and bacterial flagellin, protozoan (adjuvant) proteins, in particular Tc52 from Trypanosoma cruzi, PFTG from Trypanosoma gondii, protozoan heat shock proteins, LeIF from Leishmania spp., actin-inhibiting protein-like protein from Toxoplasma gondii, viral (adjuvant) proteins, in particular respiratory syncytial virus fusion glycoprotein (F-protein), envelope proteins from MMT virus, murine leukemia virus proteins, hemagglutinin protein of wild-type measles virus, fungal (adjuvant) proteins, in particular fungal immunomodulatory protein (FIP; LZ-8); and keyhole limpet hemocyanin (KLH), OspA; therapeutic proteins for hormone replacement therapy, in particular estrogens, progestin or progesterone, and testosterone; therapeutic proteins for reprogramming somatic cells to pluripotent or totipotent stem cells, in particular Oct-3/4, the Sox gene family (Sox1, Sox2, Sox3 and Sox15), the Klf family (Klf1, Klf2, Klf4 and Klf5), the Myc family (c-myc, L-myc and N-myc), Nanog and LIN28; and therapeutic antibodies selected from antibodies for the treatment of cancer or a tumor disease, in particular 131T-tositumomab, 3F8, 8H9, abagovomab, adecatumumab, afutuzumab, alacizumab pegol, alemtuzumab, amatuximab, AME-133v, AMG102, anatumomab, apolizumab, basiliximab, bectumomab, belimumab, bevacizumab, bivatuzumab-DM1, blinatumomab, brentuximab vedotin, cantuzumab, cantuzumab mertansine, cantuzumab ravtansine, capromab pendetide, carlumab, catumaxomab, cetuximab, citatuzumab bogatox, cixutumumab, clivatuzumab tetraxetan, CNTO328, CNTO 95, conatumumab, dacetuzumab, dalotuzumab, denosumab, denosumab, drozitumab, ecromeximab, edrecolomab, elotuzumab, elsilimomab, enavatuzumab, ensituximab, epratuzumab, ertumaxomab, etaracizumab, farletuzumab, FBTA05, ficlatuzumab, figitumumab, flanvotumab, galiximab, galiximab, ganitumab, GC1008, gemtuzumab, gemtuzumab ozogamicin, girentuximab, glemnbatumumab vedotin, GS6624, HuC242-DM4, HuHMFG1, HuN901-DM1, zevalin, icrucumab, ID09C3, indatuximab ravtansine, inotuzumab ozogamicin, intetumumab, ipilimumab, iratumumab, labetuzumab, lexatumumab, fintuzumab, lorvotuzumab mertansine, lucatumumab, lumiliximab, mapatumumab, matuzumab, MDX-060, MEDI 522, mitumomab, mogamulizumab, MORab-003, MORab-009, moxetumomab pasudotox, MT103, nacolomab, naptumomab estafenatox, narnatumab, necitumumab, nimotuzumab, olaratumab, onartuzumab, oportuzumab monatox, oregovomab, PAM4, panitumumab, patritumab, pemtumomab, pertuzumab, priliximab, racotumomab, radretumab, ramucirumab, rilotumumab, rituximab, robatumumab, samalizumab, SGN-30, SGN-40, sibrotuzumab, siltuximab, tabalumab, tacatuzumab tetraxetan, taplitumomab paptox, tenatumomab, teprotumumab, TGN1412, ticilimumab (=tremelimumab), tigatuzumab, TNX-650, tositumomab, trastuzumab, TRBS07, tremelimumab, TRU-016, tucotuzumab celmoleukin, ublituximab, urelumab, vituzumab (IMMU-106), volociximab, Votumumab, WX-G250, zalutumumab and natalizumab; antibodies for the treatment of immune disorders, in particular efalizumab, epratuzumab, etrolizumab, fontofizumab, Ixekizumab, mepolizumab, milatuzumab, pooled immunoglobulins, priliximab, rituximab, rontalizumab, ruplizumab, sarilumab, vedolizumab, visilizumab, resilizumab, adalimumab, aselizumab, atinumab, atlizumab, belimumab, besilesomab, BMS-945429, briakinumab, brodalumab, canakinumab, certofizumab pegol, erlizumab, fezakinumab, golimumab, gomiliximab, infliximab, mavrilizumab, natalizumab, ocrelizumab, odulimomab, ofatumumab, ozoralizumab, pexelizumab, rovelizumab, SBI-087, secukinumab, sirukumab, talizumab, tocilizumab, toralizumab, TRU-015, TRU-016, ustekinutnab, vepalimomab, zolimomab aritox, sifalimumab, lumiliximab, and Rho(D) immunoglobulin; antibodies for the treatment of infectious diseases, in particular, afimomab, CR6261, edobacomab, efungumab, exbivirumab, felvizumab, foravirumab, ibalizumab, libivirumab, motavizumab, nebacumab, tuvirumab, urtoxazumab, bavituximab, pagibaximab, palivizumab, panobacumab, PRO140, rafivirumab, raxibacumab, regavirumab, sevirumab, suvizumab, and tildrakizumab; antibodies for the treatment of hematological disorders, in particular abciximab, adalimumab, eculizumab, mepolizumab and milatuzumab; antibodies for immunomodulation, in particular anti-thymocyte globulin, basiliximab, siplizumab, daclizumab, gavilimomab, inolimomab, muromonab-CD3, odulimomab and siplizumab; antibodies for the treatment of diabetes, in particular gevokizumab, otelixizumab and teplizumab; antibodies for the treatment of Alzheimer's disease, in particular bapineuzumab, crenezumab, gantenerumab, ponezumab, R1450 and solanezumab; antibodies for the treatment of asthma, in particular benralizumab, enokizumab, keliximab, lebrikizwnab, omalizumab, oxelutnab, pascolizumab and tralokinwnab; antibodies for the treatment of various disorders, in particular blosozumab, CaroRx, fresolimumab, fulranumab, romosozumab, stamulumab, tanezumab, and ranibizumab; erythropoietin (EPO), granulocyte colony stimulating factor (G-CSF), α-galactosidase A, α-L-iduronidase, thyrotropin-α, N-acetylgalactosamine-4-sulfatase (rhASB), pulmozyme, tissue plasminogen activator (TPA) activase, glucocerebrosidase, interferon (IF)β-1α, interferonβ-1b, interferon-y, interferon-α, TNF-α, IL-1 to IL-36, human growth hormone (rHGH), human insulin (BHI), human chorionic gonadotropin-α, darbepoetin-α, follicle stimulating hormone (FSH) and factor VIII, antibodies and antibody derivatives such as bispecific antibodies, multispecific antibodies, ADCs, etc., for the treatment of a variety of disorders. Peptide is a compound formed by connecting α-amino acids with peptide bonds, and it is also an intermediate product of protein hydrolysis. Generally, the number of amino acids contained in a peptide is two to nine, and peptides are variously referred to according to the number of amino acids in the peptide: dipeptide, tripeptide, tetrapeptide, pentapeptide, etc., respectively. Peptides consisting of three or more amino acid molecules are known as polypeptides, which have a molecular weight of less than 10,000 Da, being capable of passing through a semipermeable membrane without being precipitated by trichloroacetic acid and ammonium sulfate. In some literature, peptides consisting of 2-10 amino acids are also known as oligopeptides (small-molecule peptides); peptides consisting of 10-50 amino acids are known as polypeptides; peptides consisting of 50 or more amino acids are known as proteins, in other words, proteins are sometimes known as polypeptides.

"Small molecule compounds" include 7-methoxypteridine, 7-methylpteridine, abacavir, abafungin, abarelix, acebutolol, acenaphthene, acetaminophen, acetanilide, acetazolamide, acetohexamide, etretinate, acrivastine, adenine, adenosine, alatrafloxacin, albendazole, albuterol, alclofenac, aldesleukin, alemtuzumab, alfuzosin, alitretinoin, allobarbital, allopurinol, all-trans retinoic acid (ATRA), aloxiprin, alprazolam, alprenolol, altretamine, amifostine, amiloride, aminoglutethimide, aminophenazone, amiodarone hydrochloride, amitriptyline, amlodipine, amobarbital, amodiaquine, amoxapine, amphetamine, amphotericin, amphotericin B, ampicillin, amprenavir, amsacrine, amyl nitrate, amobarbital, anastrozole, anrinone, anthracene, anthracycline antibiotics, aprobarbital, arsenic trioxide, asparaginase, aspirin, astemizole, atenolol, atorvastatin, atovaquone, atrazine, atropine, atropine azathioprine, auranofin, azacitidine, azapropazone, azathioprine, azintamide, azithromycin, aztreonam, baclofen, barbital, live BCG vaccine, beclamide, beclomethasone, bendroflumethiazide, benezepril, benidipine, benorilate, benperidol, bentazepam, benzamide, benzanthracene, benzathine penicillin, benzhexol hydrochloride, benznidazole, benzodiazacyclo hepta-triene, benzoic acid, bephenium hydroxynaphthoate, betamethasone, bevacizumab (atorvastatin), bexarotene, bezafibrate, bicalutamide, bifonazole, biperiden, bisacodyl, bisantrene, bleomycin, bleomycin, bortezomib, brinzolamide, bromazepam, bromocriptine mesylate, bromperidol, brotizolam, budesonide, bumetanide, bupropion, busulfan, butalbital, butamben, butenafine hydrochloride, butobarbital, butobarbital (n-butobarbital), butoconazole, butoconazole nitrate, butyl p-hydroxybenzoate, caffeine, calcidiol, calciprotriene, calcitriol, calusterone, cambendazol, camphor, camptothecin, camptothecin analogue, candesartan, capecitabine, capsaicin, captopril, carbamazepine, carbimazole, carbofuran, carboplatin, carbromal, carimazole, carmustine, cefamandole, cephazolin, cefixime, ceftazidime, cefuroxime axetil, celecoxib, cefradine, cerivastatin, cetirizine, cetuximab, chlorambucil, chloramphenicol, chlordiazepoxide, chlormethiazole, chloroquine, chlorothiazide, chlorpheniramine, chlorproguanil hydrochloride, chlorpromazine, chlorpropamide, chlorprothixene, chlorpyrifos, chlortetracycline, chlorthalidone, chlorzoxazone, cholecalciferol, cilostazol, cimetidine, cinnarizine, cinoxacin, ciprofibrate, ciprofloxacin hydrochloride, cisapride, cisplatin, citalopram, cladribine, clarithromycin, clemastine fumarate, clioquinol, clobazam, clofarabine, clofazimine, clofibrate, clomifene citrate, clomipramine, clonazepam, clopidogrel, clotiazepam, clotrimazole, clotrimazole, cloxacillin, clozapine, cocaine, codeine, colchicine, colistin, conjugated estrogen, corticosterone, cortisone, cortisone acetate, cyclizine, cyclobarbital, cyclobenzaprine, cyclobutane-spirobarbiturate, cycloethane-spirobarbiturate, cycloheptane-spirobarbiturate, cyclohexane-spirobarbiturate, cyclopentane-spirobarbiturate, cyclophosphamide, cyclopropane-spirobarbiturate, cycloserine, ciclosporin, cyproheptadine, cyproheptadine hydrochloride, cytarabine, cytosine, dacarbazine, dactinomycin, danazol, danthron, dantrolene sodium, dapsone, darbepoetin alfa, darodipine, daunorubicin, decoquinate, dehydroepiandrosterone, delavirdine, demeclocycline, denileukin, deoxycorticosterone, desoximetasone, dexamethasone, dextroamphetamine, dextrochlorpheniramine, dexfenfluramine, dexrazoxane, dextropropoxyphene, heroin, amidotrizoic acid, diazepam, diazoxide, dichlorophene, dichlorprop, diclofenac, dicoumarin, didanosine, diflunisal, digitoxin, digoxin, dihydrocodeine, dihydroequilin, dihydroergotamine mesilate, diiodohydroxyquinoline, diltiazem hydrochloride, diloxanide furoate, dimenhydrinate, dimorphoramine, dinitolmide, diosgenin, diphenoxylate hydrochloride, biphenyl, dipyridamole, dirithromycin, disopyramide, disulfiram, diuron, docetaxel, domperidone, donepezil, doxazosin, doxazosin hydrochloride, doxorubicin (neutral), doxorubicin hydrochloride, doxycycline, dromostanolone propionate, droperidol, dyphylline, echinocandin, econazole, econazole nitrate, efavirenz, ellipticine, enalapril, enlimomab, enoximone, epinephrine, an epipodophyllotoxin derivative, epirubicin, epoetin alfa, eposartan, equilenin, equilin, ergocalciferol, ergotamine tartrate, erlotinib, erythromycin, estradiol, estramustine, estriol, estrone, ethacrvnic acid, ethambutol, ethinamate, ethionamide, ethopropazine hydrochloride, ethyl 4-aminobenzoate (benzocaine), ethyl p-hydroxybenzoate, ethinyl estradiol, etodolac, etomidate, etoposide, etretinate, exemestane, felbamate, felodipine, fenbendazole, fenbuconazole, fenbufen, fenchlorphos, fenclofenac, fenfluramine, fenofibrate, fenoldepam, fenoprofen calcium, fenoxycarb, fenpiclonil, fentanyl, fenticonazole, fexofenadine, filgrastim, finasteride, flecainide acetate, floxuridine, fludarabine, fluconazole, fluconazole, flucytosine, fludioxonil, fludrocortisone, fludrocortisone acetate, flufenamic acid, flunanisone, flunarizine hydrochloride, flunisolide, flunitrazepam, fluocortolone, fluometuron, fluorene, fluorouracil, fluoxetine hydrochloride, fluoxymesterone, flupentixol decanoate, fhiphenthixol decanoate, flurazepam, flurbiprofen, fluticasone propionate, fluvastatin, folic acid, fosinopril, fosphenytoin sodium, frovatriptan, furosemide, fulvestrant, furazolidone, gabapentin, G-BHC (lindane), gefitinib, gemcitabine, gemfibrozil, gemtuzumab, glafenine, glibenclamide, gliclazide, glimepiride, glipizide, glutethimide, glibenclamide, glyceryl trinitrate (nitroglycerin), goserelin acetate, grepafloxacin, griseofulvin, guaifenesin, guanabenz acetate, guanine, halofantrine hydrochloride, haloperidol, hydrochlorothiazide, heptabarbital, heroin, hesperetin, hexachlorobenzene, hexethal, histrelin acetate, hydrocortisone, hydroflumethiazide, hydroxyurea, scopolamine, hypoxanthine, ibritumomab, ibuprofen, idarubicin, allylbutylbarbituric acid, ifosfamide, ihydroequilenin, imatinib mesylate, imipenem, indapamide, indinavir, indomethacin, indoprofen, interferon alpha-2a, interferon alpha-2b, iodamide, iopanoic acid, iprodione, irbesartan, irinotecan, isavuconazole, isocarboxazid, isoconazole, isoguanine, isoniazid, isopropyl barbiturate, isoproturon, isosorbide dinitrate, isosorbide mononitrate, isradipine, itraconazole, itraconazole, itraconazole (Itra), ivermectin, ketoconazole, ketoprofen, ketorolac, khellin, labetalol, lamivudine, lamotrigine, lanatoside C, lanosprazole, L-DOPA, leflunomide, lenalidomide, letrozole, folinic acid, leuprolide acetate, levamisole, levofloxacin, lidocaine, linuron, lisinopril, lomefloxacin, lomustine, loperamide, loratadine, lorazepam, lorefloxacin, lormetazepam, losartan mesylate, lovastatin, lisuride maleate, maprotiline hydrochloride, mazindol, mebendazole, meclozine hydrochloride, meclofenamic acid, medazepam, metildigoxin, medroxyprogesterone acetate, mefenamic acid, mefloquine hydrochloride, megestrol acetate, melphalan, mepenzolate bromide, meprobamate, meptazinol, purinethol, mesalazine, mesna, mesoridazine, mestranol, amidone, methaqualone, methocarbamol, mephenytoin, methotrexate, methoxsalen, methsuximide, methyclothiazide, methylphenidate, mephobarbital, methylparaben, methylprednisolone, methyltestosterone, methyprylon, methysergide maleate, metoclopramide, metolazone, metoprolol, metronidazole, mianserin hydrochloride, miconazole, midazolam, mifepristone, migltol, minocycline, minoxidil, mitomycin C, mitotane, mitoxantrone, mycophenolate mofetil, molindone, montelukast, morphine, moxifloxacin hydrochloride, nabumetone, nadolol, nalbuphine, nalidixic acid, nandrolone, tetracene, naphthalene, naproxen, naratriptan hydrochloride, natamycin, nelarabine, nelfinavir, nevirapine, nicardipine hydrochloride, niacinamide, niacin, acenocoumarol, nifedipine, nilutamide, nimodipine, nimorazole, nisodipine, nitrazepam, furantoin, furacillin, nizatidine, romosozumab, norethindrone, norfloxacin, norgestrel, nortriptyline hydrochloride, nystatin, estradiol, ofloxacin, olanzapine, omeprazole, omoconazole, ondansetron hydrochloride, oprelvekin, omidazole, oxaliplatin, oxamniquine, oxantel pamoate, oxaprozin, oxatomide, oxazepam, oxcarbazepine, oxfendazole, oxiconazole, oxprenolol, oxyphenbutazone, oxyphencyclimine hydrochloride, paclitaxel, palifermin, pamidronate, p-aminosalicylic acid, pantoprazole, paramethadione, paroxetine hydrochloride, pegademase, pegaspargase, pegfilgrastim, pemetrexed disodium, penicillamine, pentaerythritol tetranitrate, pentazocin, pentazocin, pentobarbital, pentobarbitone, pentostatin, pentoxifylline, perphenazine, phenazine pimozide, perylene, phenacemide, phenacetin, phenanthrene, phenindione, phenobarbitone, phenobarbital, phenolphthalein, phenoxybenzamine, phenoxybenzamine hydrochloride, phenoxymethylpenicillin, phensuximide, phenylbutazone, phenytoin, pindolol, pioglitazone, pipobroman, piroxicam, pizotifen maleate, platinum compounds, mithramycin, polyenoid, polymyxin B, porfimer sodium, posaconazole (Posa), pramipexole, prasterone, pravastatin, praziquantel, prazosin, prazosin hydrochloride, prednisolone, prednisone, primidone, probarbital, probenecid, probucol, procarbazine, prochlorperazine, progesterone, guanatol hydrochloride, promethazine, propofol, propoxur, propranolol, propyl paraben, propylthiouracil, prostaglandin, pseudoephedrine, pteridine-2-methyl-thiol, pteridine-2-thiol, pteridine-4-methyl-thiol, pteridine-4-thiol, pteridine-7-methyl-thiol, pteridine-7-thiol, pyrantel pamoate, pyrazinamide, pyrene, pyridostigmine, pyrimethamine, quetiapine, mepacrine, quinapril, quinidine, quinidine sulfate, quinine, quinine sulfate, rabeprazole sodium, ranitidine hydrochloride, rasburicase, ravuconazole, repaglinide, reposamal, reserpine, tretinoin, rifabutin, rifampin, rifapentine, rimexolone, risperidone, ritonavir, rituximab, rizatriptan benzoate, rofecoxib, ropinirole hydrochloride, rosiglitazone, saccharin, salbutamol, salicylamide, salicylic acid, saquinavir, sargramostim, butabarbital, seco-barbital, sertaconazole, sertindole, sertraline hydrochloride, simvastatin, sirolimus, sorafenib, sparfloxacin, spiramycin, spironolactone, dihydrotestosterone, stanozolol, stavudine, diethylstilbestrol, streptozocin, strychnine, sulconazole, sulconazole nitrate, sulfacetamide, sulfadiazine, sulfamerazine, sulfamethazine, sulfamethoxazole, sulfanilamide, sulfathiazole, sulindac, sulphabenzamide, sulphacetamide, sulphadiazine, sulphadoxine, sulfisoxazole, sulphamerazine, sulpha-methoxazole, sulphapyridine, sulfasalazine, sulfinpyrazone, sulpiride, sulthiame, sumatriptan succinate, sunitinib maleate, tacrine, tacrolimus, talbutal, tamoxifen citrate, tamulosin, targretin, taxane, tazarotene, telmisartan, temazepam, temozolomide, teniposide, tenoxicam, terazosin, terazosin hydrochloride, terbinafine hydrochloride, terbutaline sulfate, terconazole, terfenadine, testolactone, testosterone, tetracycline, tetrahydrocannabinol, tetroxoprim, thalidomide, thebaine, theobromine, theophylline, thiabendazole, thiamphenicol, thioguanine, thioridazine, thiotepa, thotoin, thymine, tiagabine hydrochloride, tibolone, ticlopidine, tinidazole, tioconazole, tirofiban, tizanidine hydrochloride, tolazamide, tolbutamide, tolcapone, topiramate, topotecan, toremifene, tositumomab, tramadol, trastuzumab, trazodone hydrochloride, tretinoin, triamcinolone, triamterene, triazolam, triazoles, triflupromazine, trimethoprim, trimipramine maleate, triphenylene, troglitazone, tromethamine, tropicamide, trovafloxacin, tybamate, ubidecarenone (coenzyme Q10), undecylenic acid, uracil, uramustine, uric acid, valproic acid, valrubicin, valsartan, vancomycin, venlafaxine hydrochloride, vigabatrin, vinbarbital, vinblastine, vincristine, vinorelbine, voriconazole, xanthine, zafirlukast, zidovudine, zileuton, zoledronate, zoledronic acid, zolmitriptan, zolpidem and zopiclone.

Antigens (abbreviated as Ag) refer to substances which may cause antibody production, and may be any substances capable of inducing immune response. Foreign molecules may be recognized by immunoglobulin on B cells, or treated by antigen presenting cells, combined with a major histocompatibility complex to obtain a complex to reactivate T cells and trigger a continuous immune response.

An antigenic epitope, also known as an antigenic determinant, may be a special chemical group which consists of a continuous sequence (a protein primary structure) or a discontinuous protein three-dimensional structure and determines antigenicity. Most of antigenic epitopes are present on the surface of antigenic materials, and some of the antigenic epitopes are present inside the antigenic materials, and are exposed after treatment with enzymes or other means. One natural antigenic substance may have a variety of and a plurality of determinants.

The larger the molecule of an antigen, the greater the number of the epitopes.

Tumor-associated antigens (TAA) refer to antigen molecules present on tumor cells or normal cells, including: embryonic proteins, glycoprotein antigens, squamous cell antigens, and the like, and are commonly used for clinical diagnosis of tumors. The tumor-associated antigens are not specific to the tumor cells, may be synthesized in minute amounts on the normal cells, are highly expressed when the tumor cells proliferate, and are therefore referred to as "associated antigens". Tumors derived from the same tissue type have the same tumor-associated antigens in different individuals.

Pathogenic microorganisms refer to microorganisms, or called pathogens, which may invade human bodies and cause infections and even infectious diseases. Among the pathogens, the most harmful are bacteria and viruses. The pathogenic microorganisms include prion body, fungus, bacteria, spirochete, mycoplasma, rickettsia, chlamydia, and virus. Pathogenic microorganism antigens refers to substances which are derived from the pathogens and have a function of triggering an immune response.

"Lipid encapsulation" refers to lipid nanoparticles which provide an active or a therapeutic agent (e.g., a nucleic acid (e.g., mRNA)), and comprise full encapsulation and partial encapsulation, or both. In some embodiments, the nucleic acid (e.g., mRNA) is completely encapsulated in the lipid nanoparticle.

In various embodiments, the lipid nanoparticle has an average diameter of: about 90 nm to about 600 nm, about 100 nm to about 550 nm, about 150 nm to about 500 nm, about 200 nm to about 400 nm, about 250 nm to about 300 nm, and about 200 nm to about 300 nm, and is substantially non-toxic.

### B. Lipid Compound

In some aspects, the present disclosure discloses a lipid compound that results in the selective delivery of a lipid nanoparticle comprising same to a specific organ. The lipid compound is preferably selected from one or more of ionizable anionic lipids. In some embodiments, the lipid compound is present in the composition in a molar ratio of about 5%, 10%, 15%, 20%, 22%, 24%, 26%, 28%, 30%, 32%, 34%, 36%, 38%, 40%, or 45% to about 50%, or any range that may be derived therefrom. In some embodiments, the lipid compound may be present in a molar ratio of about 5% to about 50%, about 5% to about 45%, about 10% to about 40%, about 20% to about 35%, or about 20% to about 30%.

In some embodiments, the lipid compound is selected from an ionizable anionic lipid. The ionizable anionic lipid is a small molecule with two or more C6-C24 alkyl or alkenyl or alkynyl chains. In some aspects, the present disclosure provides one or more lipids having one or more hydrophobic components and ionizable anionic groups. The ionizable anionic groups which can be used in the ionizable anionic lipid are carboxylate groups, sulfonate groups, or phosphate groups. The ionizable anionic group may be a carboxylate group. The carboxylate group may be a compound having a negative charge at a pH value of less than 5, 6, 7, 8, 9, 10, 11, 12, 13, or 14. The hydrophobic component may be one or more C6-C24 alkyl or alkenyl or alkynyl. The compound may have one hydrophobic group, two hydrophobic groups, or three hydrophobic groups.

### C Helper lipid

In some aspects of the present disclosure, a composition containing one or more helper lipids is mixed with a cationic lipid and the lipid compound of the present invention to produce a lipid nanoparticle. In some embodiments, the cationic lipid and the lipid compound of the present invention are mixed with 1, 2, 3, 4, or 5 different types of helper lipids. It is contemplated that the cationic lipid and the lipid compound of the present invention may be mixed with a single type of a variety of different helper lipids. In some embodiments, the helper lipid comprises, but is not limited to, one or more of a phospholipid, a steroid or a steroid derivative, a polymer conjugated lipid and a modified lipid.

"Phospholipid" is any lipid comprising a phosphoester group. In some embodiments, the phospholipid is a structure containing one or two long chain C6-C24 alkyl or alkenyl groups, glycerol or sphingosine, one or two phosphoester groups, and optionally a small organic molecule. In some embodiments, the small organic molecule is an amino acid, sugar, or an amino-substituted alkoxy group, such as choline or ethanolamine. In some embodiments, the phospholipid is phosphatidyl choline. In some embodiments, the phospholipid is DOPE, DSPC, DPPC, DMPC, DOPC, POPC or SM. In some embodiments, the phospholipid is distearoyl phosphatidylcholine or dioleoyl phosphatidylethanolamine.

"Steroid and the steroid derivative" comprises any steroid or steroid derivative. As used herein, in some embodiments, the term "steroid" is a class of compounds having a tetracyclic 17-carbon cyclic structure, which may further contain one or more substitutions, wherein the substitutions include alkyl, alkoxy, hydroxy, oxo and acyl or double bonds between two or more carbon atoms. In one aspect, the ring structure of the steroid comprises three fused cyclohexyl rings and a fused cyclopentyl ring. In some embodiments, the steroid derivative comprises the above ring structure with one or more non-alkyl substitutions. In some embodiments, the steroid or the steroid derivative is a sterol. In some embodiments of the present disclosure, the steroid or the steroid derivative is a cholestane or a cholestane derivative. As described above, the cholestane derivative comprises one or more non-alkyl substitutions of the above ring system. In some embodiments, the cholestane or the cholestane derivative is a cholestene or a cholestene derivative, or a sterol or a sterol derivative. In other embodiments, the cholestane or the cholestane derivative is a cholestene and a sterol or a derivative thereof.

"Polymer conjugated lipid" refers to a lipid that inhibits aggregation of lipid nanoparticles or improves the stability of lipid nanoparticles or alters the immune response or alters the circulation time *in vivo.* The polymer conjugated lipid includes, but is not limited to, a polyethylene glycol conjugated lipid, a polylactic acid conjugated lipid, a polyamide conjugated lipid, a cationic polymer conjugated lipid, a poly-sarcosine (pSar) conjugated lipid, a poly(lactic-co-glycolic acid) (PLGA) conjugated lipid, a polyamino acid conjugated lipid, a polypeptide conjugated lipid, and a polypeptoid conjugated lipid, or a mixture thereof. In some embodiments, the "polyethylene glycol conjugated lipid" refers to any lipid to which a PEG group has been connected. In some embodiments, the PEG lipid is a diglyceride, also comprising a PEG chain connected to a glycerol group. In other embodiments, the PEG lipid is a compound containing one or more C6-C24 long chain alkyl or alkenyl groups or C6-C24 fatty acid groups connected to a linker group via a PEG chain. Some non-limiting examples of the PEG lipid includes PEG-modified phosphatidylethanolamine and phosphatidic acid, PEG-conjugated ceramide, PEG-modified dialkylamine, PEG-modified 1,2-diacyloxypropane-3-amine, PEG-modified diacylglycerol, and dialkylglycerol. In some embodiments, PEG-modified distearoyl phosphatidylethanolamine or PEG-modified dimyristoyl-sn-glycerol. In some embodiments, PEG modification is measured with the molecular weight of the PEG component of the lipid. In some embodiments, the PEG used for modification has a molecular weight of about 100 to about 15,000. In some embodiments, the molecular weight is about 200 to about 500, about 400 to about 5000, about 500 to about 3000, or about 1200 to about 3000. The molecular weight of the PEG used for modification is about 100, 200, 400, 500, 600, 800, 1000, 1250, 1500, 1750, 2000, 2250, 2500, 2750, 3000, 3500, 4000, 4500, 5000, 6000, 7000, 8000, 9000, 10000 or 12500 to about 15000. "Modified lipid" includes lipids modified by small-molecule compounds, vitamins, carbohydrates, peptides, proteins, nucleic acids, lipopolysaccharides, inorganic molecules or particles, metal ions or particles, and combinations of the substances described above.

### English abbreviation list

| Abbreviation | English |
|---|---|
| siRNA | small interference RNA |
| saRNA | small activating RNA |
| samRNA | self-amplifying mRNA |
| miRNA | microRNA |
| aiRNA | asymmetric interfering RNA |
| dsRNA | Dicer-substrate RNA |
| shRNA | small hairpin RNA |
| DOTAP | 1,2-dioleoyloxy-3-trimethylammonium propane chloride |
| DOTMA | N-[1-(2,3-dioleoyloxy)propyl]-N, N,N-trimethylammonium chloride |
| DSTAP | 1,2-stearoyl-3-trimethylammonium-propane |
| DMTAP | 1,2-dimyristoyl-3-trimethylammonium-propane |
| DDA | dimethyldioctadecylammonium |
| DOBAQ | N-(4-carboxybenzyl)-N,Ndimethyl-2,3-bis (oleoyloxy) propan-1-aminium |
| SM-102 | 8-[(2-hydroxyethyl)[6-oxo-6-(undecyloxy)hexyl]amino]-octanoic acid, 1-octylnonyl ester |
| Lipid 5 | 8-[(2-hydroxyethyl)[8-(nonyloxy)-8-oxooctyl]amino-octanoic acid, 1-octylnonyl ester |
| A6 | di(dec-3-yn-1-yl) 9-((4-(dimethylamino)butanoyl)oxy)heptadecanedioate |
| DC-chol | 3β-[N-(N',N'-dimethylaminoethane)-carbamoyl]cholesterol |
| C12-200 | 1,1'-((2-(4-(2-((2-(bis(2-hydroxydodecyl)amino)ethyl)(2-hydroxydodecyl)amino)ethyl)piperazin-1-yl)ethyl)azanediyl)bis(dodecan-2-ol) |
| CKK-E12 | 3,6-bis(4-(bis(2-hydroxydodecyl)amino)butyl)piperazine-2,5-dione |
| 5A2-SC8 | 1,19-Bis[2-[2-methyl-3-(octylthio)-1-oxopropoxy]ethyl]-4,10,16-tris[3-[2-[2-methyl-3-(octylthio)-1-oxopropoxy]ethoxy]-3-oxopropyl]-4,7,10,13,16-pentaazanonadecanedioate |
| G0-C14 | N-[2-[Bis(2-hydroxytetradecyl)amino]ethyl]-16-hydroxy-14-(2-hydroxytetradecyl)-4,7-bis[3-[[2-[(2-hydroxytetradecyl)amino]ethyl]amino]-3-oxopropyl]-10-oxo-4,7,11,14-tetraazaoctacosanamide |
| OF-2 | 3,6-Bis[4-[bis[(9Z,12Z)-2-hydroxy-9,12-octadecadien-1-yl]amino]butyl]-2,5-piperazinedione |
| OF-Deg-Lin | 9,12-Octadecadienoic acid (9Z,12Z)-, 1,1' ,1 ' ' ,1 ' ' ' -[(3,6-dioxo-2,5-piperazinediyl)bis(4,1-butanediylnitrilodi-2,1-ethanediyl)] ester |
| 92-O17S | bis(2-(tetradecylthio)ethyl) 3,3'-((3-(1H-imidazol-1-yl)propyl)azanediyl)dipropionate |
| OF-C4-Deg-Lin | 2-[4-[5-[4-[bis[2-[(9Z,12Z)-octadeca-9,12-dienoyl]oxyethyl]amino]butyl]-3,6-dioxopiperazin-2-yl]butyl-[2-[(9Z,12Z)-octadeca-9,12-dienoyl]oxyethyl]amino]ethyl (9Z,12Z)-octadeca-9,12-dienoate |
| A18-iso5-2DC18 | ethyl 1-(3-(2-ethylpiperidin-1-yl)propyl)-5,5-di((Z)-heptadec-8-en-1-yl)-2,5-dihydro-1H-imidazole-2-carboxylate |
| TT3 | N1,N3,N5-tris(3-(didodecylamino)propyl)benzene-1,3,5-tricarboxamide |
| FTT5 | hexa(octan-4-yl) 9,9',9",9‴,9ʺʺ,9‴ʺ-((((benzene-1,3,5- tricarbonyl)tris(azanediyl))tris(propane-3,1-diyl))tris(azanetriyl))hexanonanoate |
| BAMEA-O16B | bis(2-(dodecyldisulfaneyl)ethyl) 3,3'-((3-methyl-9-oxo-10-oxa-13,14-dithia-3,6-diazahexacosyl)azanediyl)dipropionate |
| Vc-Lipid | (S)-2-((R)-3,4-bis(benzyloxy)-5-oxo-2,5-dihydrofuran-2-yl)-2-hydroxyethyl 6-((3-(didodecylamino)propyl)(dodecyl)amino)hexanoate |
| C14-4 | 1,1'-((2-(2-(4-(2-((2-(2-(bis(2-hydroxytetradecyl)amino)ethoxy)ethyl)(2-hydroxytetradecyl)amino)ethyl)piperazin-1 - yl)ethoxy)ethyl)azanediyl)bis(tetradecan-2-ol) |
| Lipid 14 | ((2-((4-(dimethylamino)butanoyl)oxy)ethyl)azanediyl)bis(octane-8,1-diyl) bis(2-hexyldecanoate) |
| 4A3-Cit | 2-[3-[3-[3-[bis[3-[2-[3-(3,7-dimethyloct-6-enylsulfanyl)-2-methylpropanoyl]oxyethoxy]-3-oxopropyl]amino]propyl-methylamino]propyl-[3-[2-[3-(3,7-dimethyloct-6-enylsulfanyl)-2-methylpropanoyl]oxyethoxy]-3-oxopropyl]amino]propanoyloxy]ethyl 3-(3,7-dimethyloct-6-enylsulfanyl)-2-methylpropanoate |
| ssPalmO-Phe | [4-[2-[2-[1-[2-[2-[4-[2-[2-[4-[(Z)-octadec-9-enoyl] oxyphenyl] acetyl]oxyethyl]piperidin-1-yl]ethyldisulfanyl]ethyl]piperidin-4-yl]ethoxy]-2-oxoethyl]phenyl] (Z)-octadec-9-enoate |
| DOPE | 1,2-dioleoyl-sn-glycero-3-phosphoethanolamine |
| DSPC | 1,2-distearoyl-sn-glycero-3-phosphocholine |
| DPPC | 1,2-dipalmitoyl-sn-glycero-3-phosphocholine |
| DMPC | 1,2-dimyristoyl-sn-glycero-3-phosphocholine |
| DOPC | 1,2-dioleoyl-sn-glycero-3-phosphocholine |
| POPC | 1-palmitoyl-2-oleoyl-sn-glycero-3-phosphocholine |
| SM | Sphingomyelin |
| PEG-DMG | 1,2-Dimyristoyl-rac-glycero-3-methoxypolyethylene glycol |
| PEG-DSPE | 1,2-Distearoyl-sn-glycero-3-phosphoethanolamine-Poly (ethylene glycol) |
| ASO | Antisense oligonucleotide |
| Agomir | Agonist microRNA |
| Antagomir | Antagonist microRNA |
| pSar | Poly sarcosinylated |
| PLGA | Poly lactic-co-glycolic acid |
| circRNA | Circular RNA |
| tRNA | Transfer RNA |
| cDNA | Complementary DNA |
| pDNA | Plasmid DNA |

### DETAILED DESCRIPTION

In order to more clearly describe the objects, features, and advantages of the present invention, the present invention will be described below in detail with reference to the drawings and specific embodiments, but the embodiments of the present disclosure described below in detail are intended only to illustrate the content of the present disclosure and do not constitute any limitation to the present disclosure.

### Example 1: Synthesis of Compound

The following reagents for synthesis of the compound were all AR grade and purchased from Shanghai Titan Scientific Co.,Ltd.

The synthesis method of compound 40 was as follows:

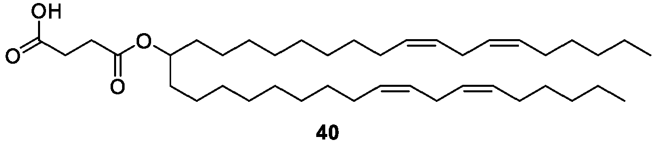

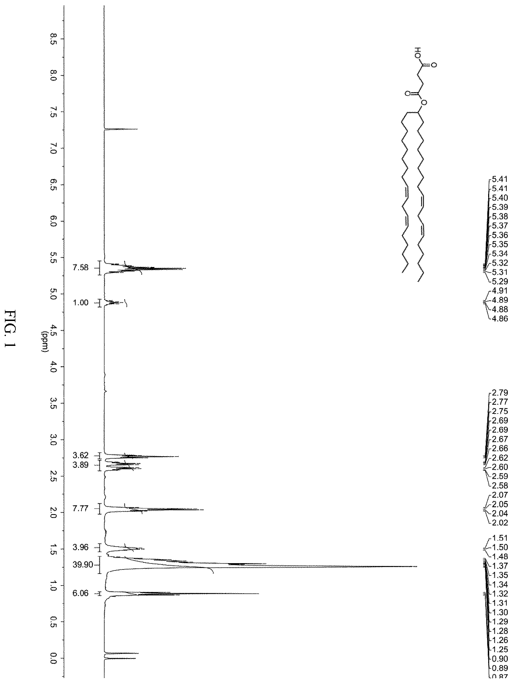

Succinic anhydride (151.4 mg, 1.51 mmol), DMAP (18.5 mg, 0.15 mmol), DLin-MeOH (400 mg, 0.76 mmol), and DCM (2 mL) were added into a 50 mL flask. After the mixture was stirred at room temperature overnight, the extent of the reaction was detected by TLC (5% MeOH/DCM). After completion of the reaction, the mixture was washed with 2 mL of water and 2 mL of saturated brine, dried over anhydrous Na₂SO₄, and filtered. The organic phase was concentrated to obtain about 500 mg of a crude product. The crude product was purified and separated using a Flash column (20 g silica gel; the mobile phases were respectively: 300 mL of n-heptane; 100 mL of 1% EtOAc + 99% n-heptane; 100 mL of 2% EtOAc + 98% n-heptane; 100 mL of 5% EtOAc + 95% *n-*heptane; 300 mL of 8% EtOAc + 92% n-heptane) to obtain a pure product (compound 40, 190 mg, 40% yield). The nuclear magnetic resonance result of compound 40 is shown in FIG. 1. 1H NMR (400 MHz, CDCl3): *δ* 5.41-5.29 (m, 8H), 4.91-4.86 (m, 1H), 2.77 (t, *J*=8Hz, 4H), 2.69-2.58 (m, 4H), 2.07-2.02 (m, 8H), 1.53-1.48 (m, 4H), 1.39-1.25 (m, 40H), 0.89 (t, *J*=8Hz, 6H).

The synthesis method of compound 43 was as follows:

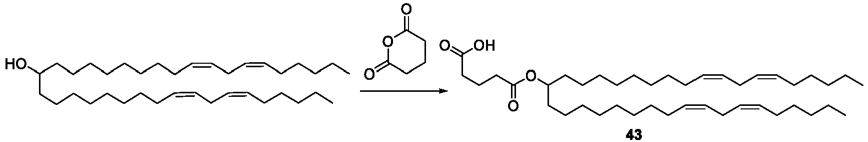

DLin-MeOH (1.5 g, 2.8 mmol), glutaric anhydride (485.5 mg, 4.3 mmol), DMAP (346.5 mg, 2.8 mmol), and DCM (5 mL) were added into a 100 mL flask. The mixture was stirred at room temperature overnight. The extent of the reaction was detected by TLC (20% EtOAc/n-heptane). After completion of the reaction, the mixture was washed with 5 mL of water and 5 mL of saturated brine, dried over anhydrous Na₂SO₄, and filtered. The organic phase was concentrated to obtain about 1.3 g of a crude product. The crude product was purified and separated using a Flash column (100 g silica gel, loading with 200 mL of *n*-heptane; the mobile phases were respectively: 200 mL of *n*-heptane; 200 mL of 0.3% EtOAc + 99.7% *n*-heptane; 200 mL of 0.5% EtOAc + 99.5% *n*-heptane; 200 mL of 1% EtOAc + 99% n-heptane; 200 mL of 2% EtOAc + 98% n-heptane; 800 mL of 3% EtOAc + 97% n-heptane) to obtain a pure product (43, 900 mg, 50% yield).

The synthesis method of compound 44 was as follows:

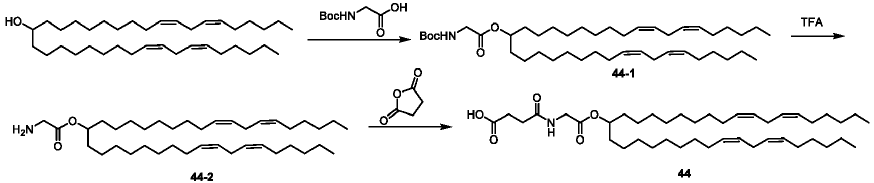

### Step 1: synthesis of compound 44-1

BOC-glycine (33 mg, 0.19 mmol), DMAP (3.4 mg, 0.028 mmol), DCC (57.8 mg, 0.28 mmol), DCM (0.75 mL), and THF (0.75 mL) were added into a 50 mL flask. The mixture was stirred for 30 min, and DLin-MeOH (150 mg, 0.28 mmol) was added to the solution. After the addition, the mixture was stirred at room temperature overnight. The extent of the reaction was detected by TLC (10% THF/*n*-heptane). After completion of the reaction, 10 mL of DCM was added for dilution, and the mixture was washed with 3 mL of water and 3 mL of saturated brine, dried over anhydrous Na₂SO₄, and filtered. The organic phase was concentrated, slurried with 2 mL of *n*-heptane, and filtered. The filtrate was concentrated to dryness to obtain a product (44-1, 50 mg, 26% yield).

### Step 2: synthesis of compound 44-2

44-1 (120 mg, 0.175 mmol) dissolved in DCM (2.0 mL) was added into a 10 mL flask, and TFA (0.4 mL) was added. After the mixture was stirred at room temperature for 2 h, the extent of the reaction was detected by TLC (20% EtOAc/n-heptane). After completion of the reaction, the reaction solution was concentrated to obtain 230 mg of a crude product. The crude product was purified and separated using a Flash column (8 g silica gel; the mobile phases were respectively: 100 mL of DCM; 100 mL of 1% MeOH + 99% DCM; 100 mL of 1.5% MeOH + 95% DCM; 300 mL of 10% MeOH + 90% DCM) to obtain a pure product (44-2, 80 mg, 78% yield).

### Step 3: synthesis of compound 44

Succinic anhydride (28 mg, 0.28 mmol), DMAP (1.7 mg, 0.014 mmol), and DCM (2 mL) were added into a 50 mL flask. After the mixture was stirred at room temperature for 30 min, 44-2 (80 mg, 0.14 mmol) was added to the reaction solution. The extent of the reaction was detected by TLC (30% EtOAc/*n*-heptane). After completion of the reaction, 10 mL of DCM was added for dilution, and the mixture was washed with 3 mL of water and 3 mL of saturated brine, dried over anhydrous Na₂SO₄, and filtered. The organic phase was concentrated to obtain about 160 mg of a crude product. The crude product was purified and separated using a Flash column (20 g silica gel; the mobile phases were respectively: 100 mL of *n*-heptane; 100 mL of 1% EtOAc + 99% *n*-heptane; 100 mL of 2% EtOAc + 98% *n*-heptane; 300 mL of 5% EtOAc + 95% n-heptane) to obtain a pure product (compound 44, 60 mg, 62% yield).

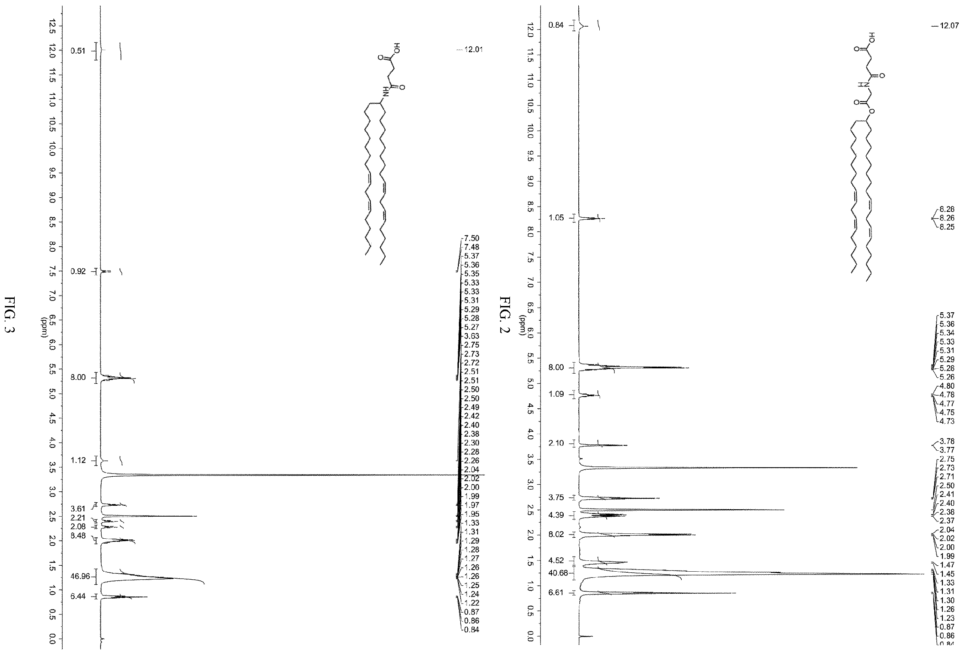

The nuclear magnetic resonance result of compound 44 is shown in FIG. 2. 1H NMR (400 MHz, *d6-*DMSO): *δ* 12.07 (s, 1H), 8.26 (t, *J*=8Hz, 1H), 5.37-5.26 (m, 8H), 4.80-4.73 (m, 1H), 3.78 (d, J=4Hz, 2H), 2.73 (t, *J*=8Hz, 4H), 2.43-2.35 (m, 4H), 2.04-1.99 (m, 8H), 1.47-1.45 (m, 4H), 1.35-1.23 (m, 40H), 0.86 (t, *J*=8Hz, 6H).

The synthesis method of compound 48 was as follows:

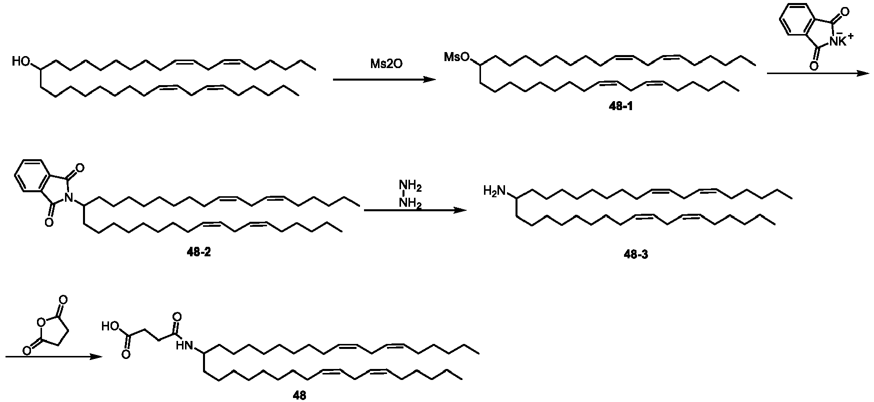

### Step 1: synthesis of compound 48-1

DLin-MeOH (750 mg, 1.4 mmol), triethylamine (287 mg, 2.8 mmol), and DCM (20 mL) were added into a 100 mL flask. Methanesulfonic anhydride (365.8 mg, 2.1 mmol) was added dropwise to the solution at 0-5 °C in an ice bath. After the dropwise addition, the mixture was stirred at 0-5 °C for 2 h, slowly heated to room temperature, and stirred overnight. The extent of the reaction was detected by TLC (5% EtOAc/*n*-heptane). After completion of the reaction, the mixture was washed with 5 mL of water and 5 mL of saturated brine, dried over anhydrous Na₂SO₄, and filtered. The organic phase was concentrated to obtain about 1.3 g of a crude product. The crude product was purified and separated using a Flash column (100 g silica gel, loading with 200 mL of *n*-heptane; the mobile phases were respectively: 200 mL of *n*-heptane; 200 mL of 0.3% EtOAc + 99.7% n-heptane; 200 mL of 1% EtOAc + 99% *n*-heptane; 600 mL of 2% EtOAc + 98% n-heptane) to obtain a pure product (48-1, 600 mg, 71% yield).

### Step 2: synthesis of compound 48-2

48-1 (1.0 g, 1.6 mmol), potassium phthalimide (0.44 g, 2.4 mmol), and DMF (15 mL) were added into a 100 mL flask. The mixture was heated to 90 °C and stirred for 16 h. The extent of the reaction was detected by TLC (10% EtOAc/*n*-heptane). After completion of the reaction, 15 mL of water was added for separation of layers, the aqueous layer was extracted with ethyl acetate (20 mL × 3), and the organic phases were combined, washed with 5 mL of saturated brine, dried over anhydrous Na₂SO₄, filtered, and concentrated to obtain about 1.6 g of a crude product. The crude product was purified and separated using a Flash column (120 g silica gel, loading with 200 mL of *n*-heptane; the mobile phases were respectively: 300 mL of *n*-heptane; 300 mL of 0.5% THF + 99.5% *n*-heptane; 600 mL of 1% THF + 99% *n*-heptane) to obtain a pure product (48-2, 800 mg, 76% yield).

### Step 3: synthesis of compound 48-3

48-2 (100 mg, 0.15 mmol), hydrazine hydrate (23.8 mg, 0.38 mmol), and absolute ethanol (0.5 mL) were added into a 100 mL flask. The mixture was heated to 80-85 °C and stirred for 4 h. The extent of the reaction was detected by TLC (10% THF/*n*-heptane). After completion of the reaction, the mixture was purified by Pre-TLC (*n*-heptane/THF = 10/1) to obtain a product (48-3, 60 mg, 80% yield).

### Step 4: synthesis of compound 48

Succinic anhydride (40.1 mg, 0.4 mmol), DMAP (4.8 mg, 0.04 mmol), and DCM (1 mL) were added into a 50 mL flask. After the mixture was stirred at room temperature for 30 min, 48-3 (100 mg, 0.2 mmol) was added to the reaction solution. The extent of the reaction was detected by TLC (30% EtOAc/*n*-heptane). After completion of the reaction, 10 mL of DCM was added for dilution, and the mixture was washed with 5 mL of water and 5 mL of saturated brine, dried over anhydrous Na₂SO₄, and filtered. The organic phase was concentrated to obtain about 100 mg of a crude product. The crude product was purified and separated using a Flash column (20 g silica gel; the mobile phases were respectively: 100 mL of *n*-heptane; 100 mL of 1% EtOAc + 99% *n*-heptane; 100 mL of 2% EtOAc + 98% *n*-heptane; 100 mL of 5% EtOAc + 95% *n*-heptane; 300 mL of 8% EtOAc + 92% *n-*heptane) to obtain a pure product (compound 48, 60 mg, 48% yield).

The nuclear magnetic resonance result of compound 48 is shown in FIG. 3. 1H NMR (400 MHz, *d6*-DMSO): *δ* 12.01 (s, 1H), 7.49 (d, *J*=8Hz, 1H), 5.37-5.27 (m, 8H), 3.63 (m, 1H), 2.73 (t, *J*=8Hz, 4H), 2.40 (t, *J*=8Hz, 2H), 2.28 (t, *J*=8Hz, 2H), 2.04-1.95 (m, 8H), 1.33-1.22 (m, 47H), 0.86 (t, *J*=8Hz, 6H).

The synthesis method of compound 70 was as follows:

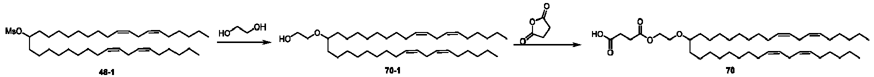

### Step 1: synthesis of compound 70-1

60% sodium hydride (25.8 mg, 0.64 mmol) was added into a 100 mL flask, ethylene glycol (1 mL) was slowly added dropwise, and a mixture of 48-1 (100 mg, 0.16 mmol) and ethylene glycol (0.5 mL) was added dropwise.

The mixture was heated to 65 °C and stirred for 2 h. DMF (2 mL) was added, and then the mixture was heated to 90 °C and stirred for 16 h. The extent of the reaction was detected by TLC (20% EtOAc/*n*-heptane). After completion of the reaction, 5 mL of water was added for separation of layers, the aqueous layer was extracted with ethyl acetate (10 mL × 3), and the organic phases were combined, washed with 5 mL of saturated brine, dried over anhydrous Na₂SO₄, filtered, and concentrated to obtain about 300 mg of a crude product. The crude product was purified and separated using a Flash column (20 g silica gel, loading with 100 mL of *n*-heptane; the mobile phases were respectively: 100 mL of *n*-heptane; 100 mL of 1% EtOAc + 99% n-heptane; 100 mL of 3% EtOAc + 97% *n*-heptane; 100 mL of 5% EtOAc + 95% n-heptane; 500 mL of 10% EtOAc + 90% *n*-heptane) to obtain a pure product (70-1, 60 mg, 66% yield).

### Step 2: synthesis of compound 70

Succinic anhydride (63.3 mg, 0.62 mmol), DMAP (7.6 mg, 0.062 mmol), and DCM (2 mL) were added into a 50 mL flask. After the mixture was stirred at room temperature for 30 min, 70-1 (180 mg, 0.31 mmol) was added to the reaction solution. The extent of the reaction was detected by TLC (30% EtOAc/*n*-heptane). After completion of the reaction, 10 mL of DCM was added for dilution, and the mixture was washed with 5 mL of water and 5 mL of saturated brine, dried over anhydrous Na₂SO₄, and filtered. The organic phase was concentrated to obtain about 180 mg of a crude product. The crude product was purified and separated using a Flash column (20 g silica gel; the mobile phases were respectively: 100 mL of n-heptane; 100 mL of 1% EtOAc + 99% *n*-heptane; 100 mL of 2% EtOAc + 98% *n*-heptane; 100 mL of 5% EtOAc + 95% *n*-heptane; 300 mL of 8% EtOAc + 92% *n*-heptane) to obtain a pure product (compound 70, 100 mg, 48% yield).

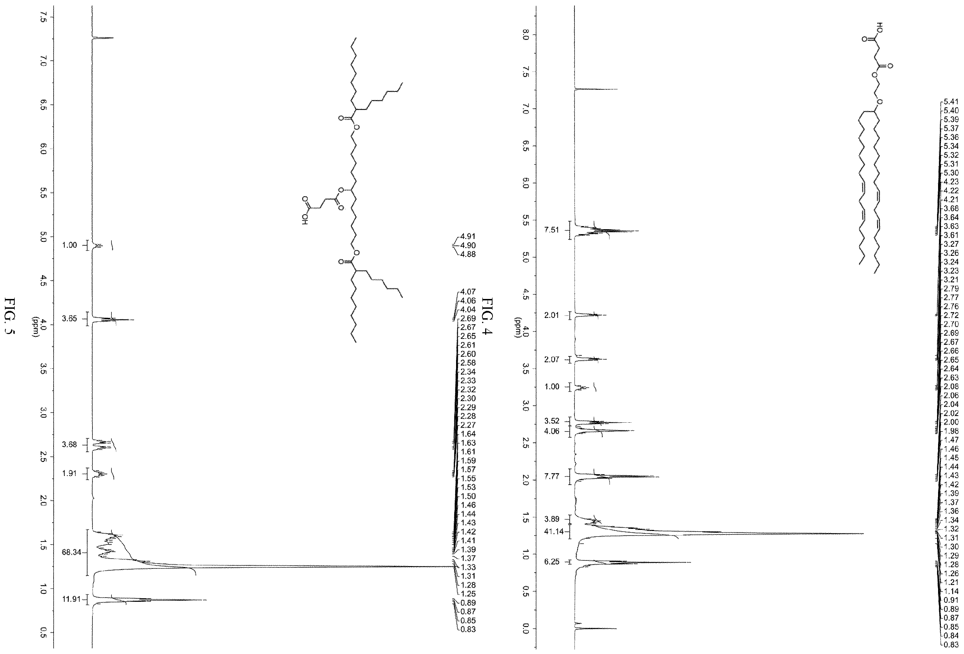

The nuclear magnetic resonance result of compound 70 is shown in FIG. 4. 1H NMR (400 MHz, CDCl3): *δ* 5.41-5.30 (m, 8H), 4.22 (t, *J*=8Hz, 2H), 3.63 (t, *J*=8Hz, 2H), 3.27-3.21 (m, 1H), 2.77 (t, *J*=8Hz, 4H), 2.72-2.63 (m, 4H), 2.08-1.98 (m, 8H), 1.47-1.42 (m, 4H), 1.37-1.21 (m, 40H), 0.89 (t, *J*=8Hz, 6H).

The synthesis method of compound 130 was as follows:

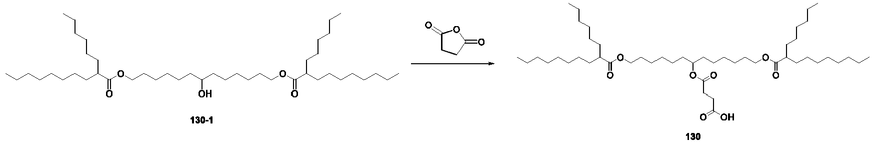

130-1 (200 mg, 0.28 mmol, 1.0 eq), DMAP (6.89 mg, 0.056 mmol, 0.2 eq), and succinic anhydride (56.44 mg, 0.56 mmol, 2.0 eq) were added into a 25 mL flask and dissolved in DCM (2 mL). The mixture was stirred at room temperature overnight. The extent of the reaction was detected by TLC (35% EtOAc/*n*-heptane). After completion of the reaction, the mixture was washed with 2 mL of water and 2 mL of saturated brine, and the organic phase was dried over anhydrous Na₂SO₄, filtered, and concentrated to obtain about 500 mg of a crude product. The crude product was purified and separated using a Flash column (30 g silica gel, loading with 100 mL of *n*-heptane; the mobile phases were respectively: 100 mL of *n*-heptane; 100 mL of 1% EtOAc + 99% *n-*heptane; 100 mL of 2% EtOAc + 98% *n*-heptane; 100 mL of 3% EtOAc + 97% *n*-heptane; 300 mL of 10% EtOAc + 90% *n*-heptane) to obtain a pure product (compound 130, 30 mg, 13% yield).

The nuclear magnetic resonance result of compound 130 is shown in FIG. 5. 1H NMR (400 MHz, CDCl₃): *δ* 4.91-4.88 (m, 1H), 4.06 (t, *J*=8Hz, 1H), 2.69-2.58 (m, 4H), 2.34-1.27 (m, 2H), 1.64-1.25 (m, 68H), 1.65-0.85 (m, 48H), 0.89-0.83 (m, 12H).

The synthesis method of compound 131 was as follows:

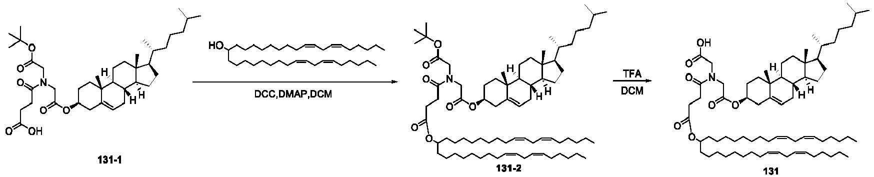

### Step 1: synthesis of compound 131-2

131-1 (746.3 mg, 1.1 mmol) dissolved in DCM (10 mL) was added into a 50 mL flask, and DMAP (57.7 mg, 0.47 mmol) and DCC (234.1 mg, 1.1 mmol) were added. After the mixture was stirred at room temperature for 30 min, Dlin-MEOH (500 mg, 0.95 mmol) was added to the solution, and the mixture was stirred at room temperature overnight. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 800 mg of a crude product. The crude product was purified and separated using a Flash column (12 g silica gel; the mobile phases were respectively: 300 mL of *n*-heptane; 200 mL of 1% EtOAc + 99% *n*-heptane; 200 mL of 5% EtOAc + 95% *n*-heptane; 300 mL of 8% EtOAc + 92% *n*-heptane) to obtain a pure product (131-2, 550 mg, 50% yield).

### Step 2: synthesis of compound 131

131-2 (550 mg, 0.47 mmol) dissolved in DCM (5 mL) was added into a 50 mL flask, and TFA (2 mL) was added. After the mixture was stirred at room temperature for 2 h, the extent of the reaction was detected by TLC (20% EtOAc/*n*-heptane). After completion of the reaction, the reaction solution was concentrated, and MTBE (20 mL) was added and dissolved. The pH was adjusted to 8-9 with 10% NaHCO₃ solution, and then the pH was adjusted to 1-2 with 1 M diluted hydrochloric acid. The mixture was stirred for 20 min, and separation of layers was performed. The aqueous phase was extracted with 20 mL of MTBE, and the organic phases were combined, dried over anhydrous Na₂SO₄, filtered, and concentrated to obtain 300 mg of a crude product. The crude product was purified and separated using a Flash column (8 g silica gel; the mobile phases were respectively: 200 mL of n-heptane; 100 mL of 5% EtOAc + 95% n-heptane; 100 mL of 10% EtOAc + 90% n-heptane; 100 mL of 20% EtOAc + 80% n-heptane; 200 mL of 30% EtOAc + 70% n-heptane) to obtain a pure product (compound 131, 100 mg, 19% yield).

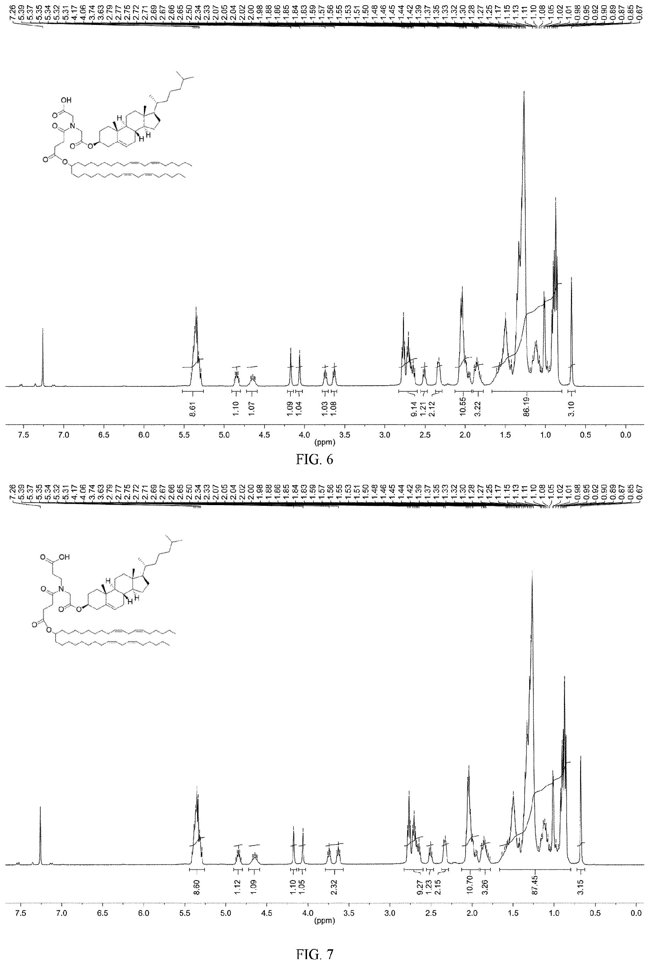

The nuclear magnetic resonance result of compound 131 is shown in FIG. 6. 1H NMR (400 MHz, CDCl₃): *δ* 5.41-5.29 (m, 9H), 4.88-4.81 (m, 1H), 4.67-4.62 (m, 1H), 4.17 (s,1H), 4.06 (s,1H), 3.74 (t, 1H, *J*=8Hz), 3.63 (t, 1H, *J*=4Hz), 2.79-2.63 (m, 9H), 2.51 (t, 1H, *J*=8Hz), 2.34-2.33 (m,2H), 2.07-1.94 (m,10H), 1.88-1.80 (m,3H), 1.61-0.85 (m,77H), 0.67 (s,3H).

The synthesis method of compound 132 was as follows:

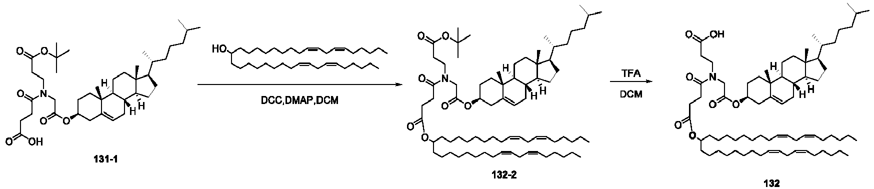

### Step 1: synthesis of compound 132-2

131-1 (762.2 mg, 1.1 mmol) dissolved in DCM (10 mL) was added into a 50 mL flask, and DMAP (57.7 mg, 0.47 mmol) and DCC (234.1 mg, 1.1 mmol) were added. After the mixture was stirred at room temperature for 30 min, Dlin-MEOH (500 mg, 0.95 mmol) was added to the solution, and the mixture was stirred at room temperature overnight. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 930 mg of a crude product. The crude product was purified and separated using a Flash column (12 g silica gel; the mobile phases were respectively: 300 mL of *n*-heptane; 200 mL of 1% EtOAc + 99% *n*-heptane; 200 mL of 5% EtOAc + 95% *n*-heptane; 300 mL of 8% EtOAc + 92% *n*-heptane) to obtain a pure product (132-2, 670 mg, 60% yield).

### Step 2: synthesis of compound 132

132-2 (610 mg, 0.52 mmol) dissolved in DCM (7 mL) was added into a 50 mL flask, and TFA (3 mL) was added. After the mixture was stirred at room temperature for 2 h, the extent of the reaction was detected by TLC (60% EtOAc/n-heptane). After completion of the reaction, the reaction solution was concentrated, and MTBE (20 mL) was added and dissolved. The pH was adjusted to 8-9 with 10% NaHCO₃ solution, and then the pH was adjusted to 1-2 with 1 M diluted hydrochloric acid. The mixture was stirred for 20 min, and separation of layers was performed. The aqueous phase was extracted with 20 mL of MTBE, and the organic phases were combined, dried over anhydrous Na₂SO₄, filtered, and concentrated to obtain 300 mg of a crude product. The crude product was purified and separated using a Flash column (8 g silica gel; the mobile phases were respectively: 200 mL of n-heptane; 100 mL of 5% EtOAc + 95% n-heptane; 100 mL of 10% EtOAc + 90% n-heptane; 100 mL of 20% EtOAc + 80% *n*-heptane; 200 mL of 30% EtOAc + 70% *n*-heptane; 200 mL of 50% EtOAc + 50% *n*-heptane) to obtain a pure product (compound 132, 110 mg, 18% yield).

The nuclear magnetic resonance result of compound 132 is shown in FIG. 7. 1H NMR (400 MHz, CDCl₃): *δ* 5.41-5.29 (m, 9H), 4.88-4.81 (m, 1H), 4.67-4.62 (m, 1H), 4.17 (s,1H), 4.06 (s,1H), 3.74 (t, 1H, *J*=8Hz), 3.63 (t, 1H, *J*=8Hz), 2.79-2.63 (m, 9H), 2.50 (t, 1H, *J*=4Hz), 2.34-2.33 (m,2H), 2.07-1.94 (m,10H), 1.88-1.78 (m,3H), 1.64-0.85(m,79H), 0.67 (s,3H).

The synthesis method of compound 133 was as follows:

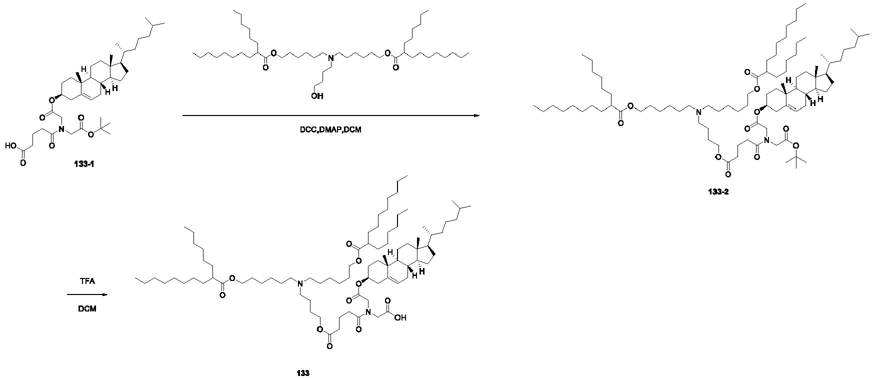

### Step 1: synthesis of compound 133-2

133-1 (526 mg, 0.78 mmol) dissolved in DCM (5 mL) was added into a 50 mL flask, and DMAP (39.7 mg, 0.33 mmol) and DCC (160.9 mg, 0.78 mmol) were added. After the mixture was stirred at room temperature for 30 min, ALC-0315 (500 mg, 0.65 mmol) was added to the solution, and the mixture was stirred at room temperature overnight. The extent of the reaction was detected by TLC (20% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 820 mg of a crude product. The crude product was purified and separated using a Flash column (15 g silica gel; the mobile phases were respectively: 300 mL of DCM; 200 mL of 1% MeOH + 99% DCM; 200 mL of 1.5% MeOH + 98.5% DCM; 200 mL of 2% MeOH + 98% DCM; 200 mL of 3% MeOH + 97% DCM) to obtain a pure product (133-2, 500 mg, 54% yield).

### Step 2: synthesis of compound 133

133-2 (500 mg, 0.35 mmol) dissolved in DCM (9 mL) was added into a 50 mL flask, and TFA (4 mL) was added. After the mixture was stirred at room temperature for 2 h, the extent of the reaction was detected by TLC (20% MeOH/DCM). After completion of the reaction, the reaction solution was concentrated, and MTBE (20 mL) was added and dissolved. The pH was adjusted to 8-9 with 10% NaHCO₃ solution, and then the pH was adjusted to 1-2 with 1 M diluted hydrochloric acid. The mixture was stirred for 20 min, and separation of layers was performed. The aqueous phase was extracted with 20 mL of MTBE, and the organic phases were combined, dried over anhydrous Na₂SO₄, filtered, and concentrated to obtain 480 mg of a crude product. The crude product was purified and separated using a Flash column (8 g silica gel; the mobile phases were respectively: 300 mL of DCM; 200 mL of 1% MeOH + 99% DCM; 200 mL of 1.5% MeOH + 98.5% DCM; 200 mL of 2% MeOH + 98% DCM; 200 mL of 3% MeOH + 97% DCM; 200 mL of 5% MeOH + 95% DCM) to obtain a pure product (compound 133, 200 mg, 42% yield).

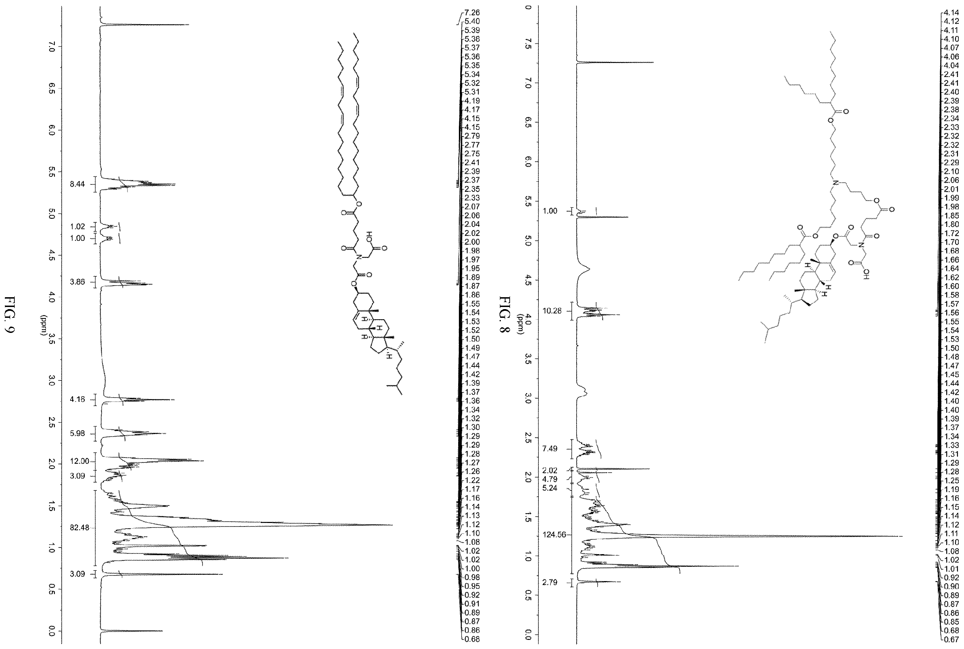

The nuclear magnetic resonance result of compound 133 is shown in FIG. 8. 1H NMR (400 MHz, CDCl₃): *δ* 5.37-5.34 (m, 1H), 4.14-4.04 (m, 10H), 2.41-2.29 (m, 7H), 2.01 (s, 2H), 2.06-1.98(m, 5H), 1.85-0.85 (m, 121H), 0.67(s,3H).

The synthesis method of compound 134 was as follows:

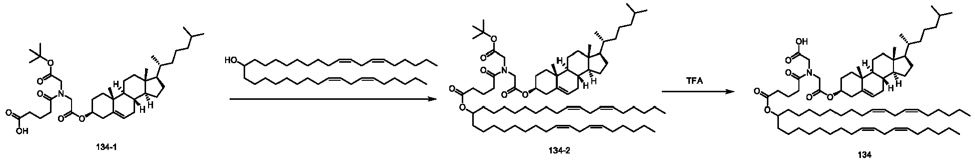

### Step 1: synthesis of compound 134-2

134-1 (500 mg, 0.74 mmol) dissolved in DCM (5 mL) was added into a 50 mL flask, and DMAP (18.2 mg, 0.15 mmol) and DCC (230.3 mg, 1.12 mmol) were added. After the mixture was stirred at room temperature for 30 min, Dlin-MeOH (393.6 mg, 0.74 mmol) was added to the solution, and the mixture was stirred at room temperature overnight. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 700 mg of a crude product. The crude product was purified and separated using a Flash column (12 g silica gel; the mobile phases were respectively: 300 mL of n-heptane; 200 mL of 1% EtOAc + 99% n-heptane; 200 mL of 5% EtOAc + 95% n-heptane; 300 mL of 8% EtOAc + 92% n-heptane) to obtain a pure product (134-2, 500 mg, 57% yield). ¹H NMR (400 MHz, CDCl₃): δ 5.41-5.29 (m, 8H), 4.86-4.83 (m, 1H), 4.72-4.60 (m, 1H), 4.13-4.03 (m, 4H), 2.79-2.75 (m, 4H), 2.39-2.32 (m, 6H),1.07-1.78 (m, 16H), 1.65-0.85 (m, 92H), 0.68(s,3H).

### Step 2: synthesis of compound 134

134-2 (500 mg, 0.42 mmol) dissolved in DCM (12 mL) was added into a 50 mL flask, and TFA (4 mL) was added. After the mixture was stirred at room temperature for 2 h, the extent of the reaction was detected by TLC (20% EtOAc/n-heptane). After completion of the reaction, the reaction solution was concentrated, and MTBE (20 mL) was added and dissolved. The pH was adjusted to 8-9 with 10% NaHCO₃ solution, and then the pH was adjusted to 1-2 with 1 M diluted hydrochloric acid. The mixture was stirred for 20 min, and separation of layers was performed. The aqueous phase was extracted with MTBE (20 mL), and the organic phases were combined, dried over anhydrous Na₂SO₄, filtered, and concentrated to obtain 410 mg of a crude product. The crude product was purified and separated using a Flash column (8 g silica gel; the mobile phases were respectively: 200 mL of n-heptane; 100 mL of 5% EtOAc + 95% n-heptane; 100 mL of 10% EtOAc + 90% n-heptane; 100 mL of 20% EtOAc + 80% n-heptane; 200 mL of 50% EtOAc + 50% n-heptane) to obtain a pure product (compound 134, 200 mg, 42% yield).

The nuclear magnetic resonance result of compound 134 is shown in FIG. 9. 1H NMR (400 MHz, CDCl₃): δ 5.41-5.29 (m, 8H), 4.86-4.83 (m, 1H), 4.72-4.68 (m, 1H), 4.19-4.15 (m, 4H), 2.79-2,72(m, 4H), 2.41-2.33 (m, 6H), 2.07-1.85 (m, 16H), 1.58-0.86 (m, 83H), 0.68(s,3H).

The synthesis method of compound 135 was as follows:

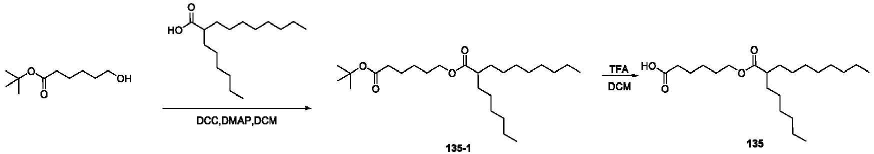

### Step 1: synthesis of compound 135-1

DMAP (2.5 g, 20.1 mmol), DCC (4.1 g, 20.1 mmol), and DCM (50 mL) were added into a 250 mL flask, and the mixture was stirred until it became clear. 2-Hexyldecanoic acid (5.13 g, 20.1 mmol) was added, and the mixture was stirred at room temperature for 30 min. tert-Butyl 6-hydroxyhexanoate (3.6 g, 19.1 mmol) was then added in one batch, and the mixture was stirred at room temperature overnight. The extent of the reaction was detected by TLC (30% n-heptane/DCM). After completion of the reaction, the reaction solution was filtered to remove solids, and the filtrate was washed with 15 mL of water and 15 mL of saturated brine, dried over anhydrous Na₂SO₄, and filtered. The organic phase was concentrated to obtain about 9.3 g of a crude product. The crude product was purified and separated using a Flash column (80 g silica gel, loading with 100 mL of PE; the mobile phases were respectively: 300 mL of n-heptane; 300 mL of 10% DCM + 90% n-heptane; 300 mL of 20% DCM + 60% n-heptane) to obtain a pure product (135-1, 7 g, 86% yield).

### Step 2: synthesis of compound 135

135-1 (600 mg, 9.7 mmol) dissolved in DCM (12 mL) was added into a 50 mL flask, and TFA (4 mL) was added. After the mixture was stirred at room temperature for 2 h, the extent of the reaction was detected by TLC (0.5% MeOH/DCM). After completion of the reaction, the reaction solution was concentrated, and MTBE (20 mL) was added and dissolved. The pH was adjusted to 8-9 with 10% NaHCO₃ solution, and then the pH was adjusted to 1-2 with 1 M diluted hydrochloric acid. The mixture was stirred for 20 min, and separation of layers was performed. The aqueous phase was extracted with 20 mL of MTBE, and the organic phases were combined, dried over anhydrous Na₂SO₄, filtered, and concentrated to obtain 620 mg of a crude product. The crude product was purified and separated using a Flash column (10 g silicagel, loading with 50 mL of DCM; the mobile phases were respectively: 100 mL of DCM; 100 mL of 1% MeOH + 99% DCM; 100 mL of 2% MeOH + 98% DCM; 100 mL of 3% MeOH + 97% DCM; 100 mL of 4% MeOH + 96% DCM) to obtain a pure product (compound 135, 410 mg, 78% yield).

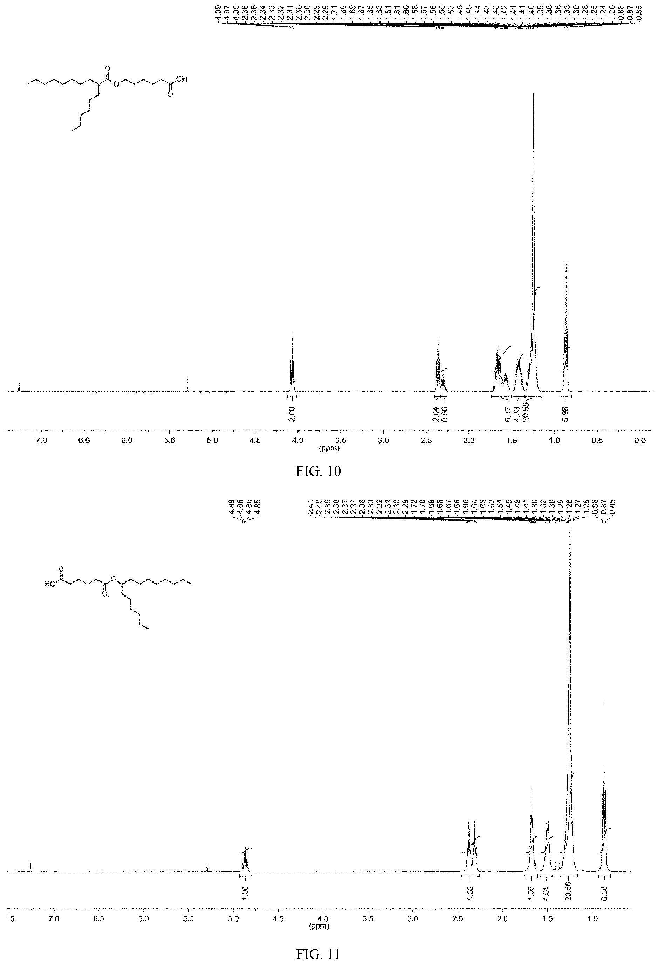

The nuclear magnetic resonance result of compound 135 is shown in FIG. 10. 1H NMR (400 MHz, CDCl₃): *δ* 4.07 (t, J=8Hz, 2H), 2.36 (t, J=8Hz, 2H), 2.33-2,28 (m, 1H), 1,71-1.53 (m, 6H), 1.46-1.36 (m, 4H), 1.33-1.20 (m, 20H), 0.87 (t, J=8Hz, 6H).

The synthesis method of compound 136 was as follows:

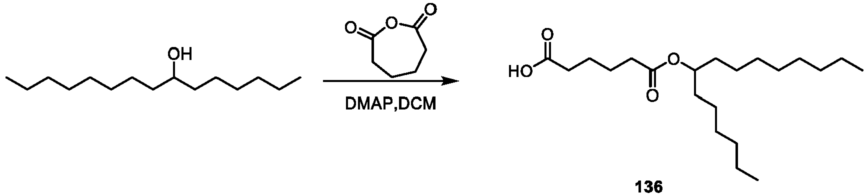

Pentadecan-7-ol (1.9 g, 8.32 mmol), DMAP (1.02 g, 8.32 mmol), and DCM (19 mL) were added into a 100 mL flask, and the mixture was stirred until it became clear. Adipic anhydride (1.6 g, 12.5 mmol) was added, and the mixture was stirred at room temperature for 2 h. The extent of the reaction was detected by TLC (30% EtOAc/PE). After completion of the reaction, the mixture was washed with 5 mL of water and 5 mL of saturated brine, dried over anhydrous Na₂SO₄, and filtered. The organic phase was concentrated to obtain about 3.1 g of a crude product. The crude product was purified and separated using a Flash column (60 g silica gel, loading with 200 mL of PE; the mobile phases were respectively: 300 mL of 1% EtOAc + 99% n-heptane; 300 mL of 2% EtOAc + 98% n-heptane; 300 mL of 5% EtOAc + 95% n-heptane; 1000 mL of 10% EtOAc + 90% n-heptane) to obtain a pure product (compound 136, 1.2 g, 40% yield).

The nuclear magnetic resonance result of compound 136 is shown in FIG. 11. 1H NMR (400 MHz, CDCl₃): *δ* 4.89-4.85 (m, 1H), 2.41-2.29 (m, 4H), 1.72-1.63 (m, 4H), 1.52-1.48 (m, 4H), 1.32-1.25 (m, 20H), 0.87 (t, J=4Hz, 6H).

The synthesis method of compound 137 was as follows:

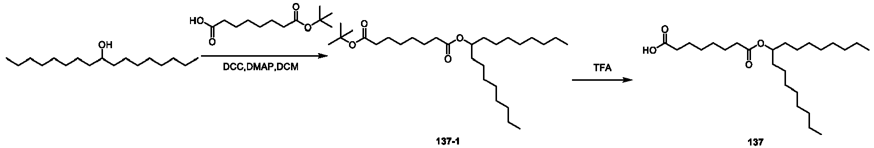

### Step 1: synthesis of compound 137-1

DMAP (500.2 mg, 4.1 mmol), DCC (1.69 g, 8.2 mmol), and DCM (20 mL) were added into a 250 mL flask, and the mixture was stirred until it became clear. 8-(*tert*-Butoxy)-8-oxooctanoic acid (1.9 g, 8.2 mmol) was added, and the mixture was stirred at room temperature for 30 min. 9-Heptadecanol (2.1 g, 8.2 mmol) was then added in one batch, and the mixture was stirred at room temperature overnight. The extent of the reaction was detected by TLC (10% EtOAc/n-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to obtain about 3.2 g of a crude product. The crude product was purified and separated using a Flash column (30 g silica gel, loading with 200 mL of n-heptane; the mobile phases were respectively: 200 mL of 0.5% EtOAc + 99.5% n-heptane; 200 mL of 1% EtOAc + 99% n-heptane; 1000 mL of 2% EtOAc + 98% n-heptane) to obtain a pure product (137-1, 2.1 g, 55% yield).

### Step 2: synthesis of compound 137

137-1 (1.2 g, 2.56 mmol) dissolved in DCM (8 mL) was added into a 100 mL flask, and TFA (4 mL) was added. After the mixture was stirred at room temperature for 2 h, the extent of the reaction was detected by TLC (5% MeOH/DCM). After completion of the reaction, the reaction solution was concentrated, and MTBE (20 mL) was added and dissolved. The pH was adjusted to 8-9 with 10% NaHCO₃ solution, and then the pH was adjusted to 1-2 with 1 M diluted hydrochloric acid. The mixture was stirred for 20 min, and separation of layers was performed. The aqueous phase was extracted with 50 mL of MTBE, and the organic phases were combined, dried over anhydrous Na₂SO₄, filtered, and concentrated to obtain 1.5 g of a crude product. The crude product was purified and separated using a Flash column (100 g silica gel, loading with 50 mL of n-heptane; the mobile phases were respectively: 300 mL of 1% EtOAc + 99% n-heptane; 200 mL of 1.5% EtOAc + 98.5% n-heptane; 1000 mL of 2% EtOAc + 98% n-heptane) to obtain a pure product (compound 137, 800 mg, 80% yield).

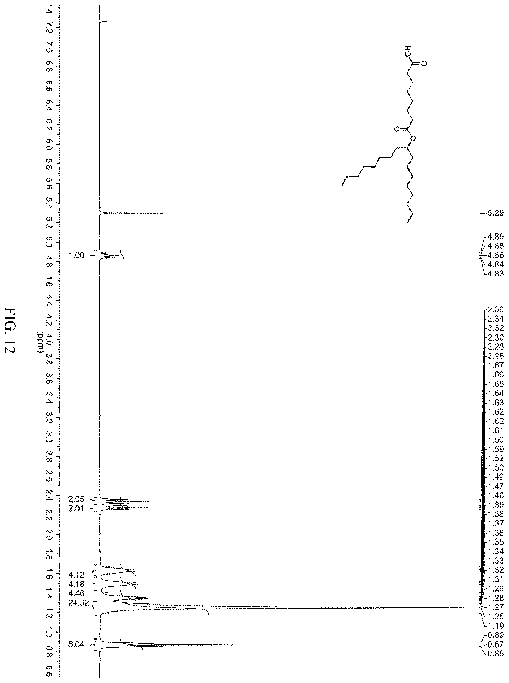

The nuclear magnetic resonance result of compound 137 is shown in FIG. 12. 1H NMR (400 MHz, CDCl₃): *δ* 4.89-4.83 (m, 1H), 2.34 (t, *J*=8Hz, 2H), 2.28 (t, *J*=8Hz, 2H), 1.67-1.59 (m, 4H), 1.52-1.47 (m, 4H), 1.40-1.32 (m, 4H), 1.31-1.19 (m, 24H), 0.87 (t, *J*=8Hz, 6H).

DODMA, DSPC, cholesterol, PEG2000-DMG, etc. used in the following examples were all from AVT (Shanghai) Pharmaceutical Tech Co., Ltd., and Luc-mRNA (luciferase mRNA) was from Shanghai Hongene Biotech Corporation.

Example 2: Preparation of Lipid Nanoparticle TMF7 (Containing 20% Compound 40):
1. DODMA (cationic lipid), DSPC (helper lipid), cholesterol (helper lipid), PEG2000-DMG (helper lipid), and compound 40 (lipid compound of the present disclosure) were dissolved in ethanol to obtain an oil phase (ethanol phase) stock solution in molar ratios of 37.0%, 7.5%, 34.2%, 1.3%, and 20.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to obtain TMF7 with a weight ratio of total lipids to mRNA of 25.9:1. The sample was then concentrated with an ultrafiltration tube having a molecular weight cutoff of 100 kd, washed with PBS buffer with a pH of 7.4 and filtered, and finally the filtrate was buffer-exchanged with 120 mg/mL sucrose PBS buffer to obtain a final sample.

Example 3: Preparation of Lipid Nanoparticle TMF8 (Containing 20% Compound 44):
1. DODMA (cationic lipid), DSPC (helper lipid), cholesterol (helper lipid), PEG2000-DMG (helper lipid), and compound 44 (lipid compound of the present disclosure) were dissolved in ethanol to obtain an oil phase (ethanol phase) stock solution in molar ratios of 37.0%, 7.5%, 34.2%, 1.3%, and 20.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to obtain TMF8 with a weight ratio of total lipids to mRNA of 26.3:1. The sample was then concentrated with an ultrafiltration tube having a molecular weight cutoff of 100 kd, washed with PBS buffer with a pH of 7.4 and filtered, and finally the filtrate was buffer-exchanged with 120 mg/mL sucrose PBS buffer to obtain a final sample.

Example 4: Preparation of Lipid Nanoparticle TMF9 (Containing 20% Compound 70):
1. DODMA (cationic lipid), DSPC (helper lipid), cholesterol (helper lipid), PEG2000-DMG (helper lipid), and compound 70 (lipid compound of the present disclosure) were dissolved in ethanol to obtain an oil phase (ethanol phase) stock solution in molar ratios of 37.0%, 7.5%, 34.2%, 1.3%, and 20.0%, respectively.
2. The luciferase-expressing mRNA stock was diluted to 0.09 mg/mL with pH 4 citrate buffer to obtain an aqueous phase.
3. The oil phase (ethanol phase) containing the mixture of five lipids and the aqueous phase containing mRNA were rapidly mixed in a volume ratio of 1:3 to obtain TMF9 with a weight ratio of total lipids to mRNA of 26.3:1. The sample was then concentrated with an ultrafiltration tube having a molecular weight cutoff of 100 kd, washed with PBS buffer with a pH of 7.4 and filtered, and finally the filtrate was buffer-exchanged with 120 mg/mL sucrose PBS buffer to obtain a final sample.

Example 5: Preparation of Lipid Nanoparticle TMF100 (Containing 38.5% Compound 40):
1. DODMA (cationic lipid), DSPC (helper lipid), compound 40 (lipid compound of the present disclosure), and PEG2000-DMG (helper lipid) were dissolved in ethanol to obtain an oil phase (ethanol phase) stock solution in molar ratios of 50%, 10%, 38.5%, and 1.5%, respectively.
2. The luciferase-expressing mRNA stock was diluted to 0.3 mg/mL with pH 4 citrate buffer to obtain an aqueous phase.
3. The oil phase (ethanol phase) containing the mixture of four lipids and the aqueous phase containing mRNA were rapidly mixed in a volume ratio of 1:3 to obtain TMF100 with a weight ratio of total lipids to mRNA of 27.1:1. The sample was then concentrated with an ultrafiltration tube having a molecular weight cutoff of 100 kd, washed with PBS buffer with a pH of 7.4 and filtered, and finally the filtrate was buffer-exchanged with 120 mg/mL sucrose PBS buffer to obtain a final sample.

Example 6: Preparation of Lipid Nanoparticle TMF107 (Containing 38.5% Compound 134):
1. DODMA (cationic lipid), DSPC (helper lipid), compound 134 (lipid compound of the present disclosure), and PEG2000-DMG (helper lipid) were dissolved in ethanol to obtain an oil phase (ethanol phase) stock solution in molar ratios of 50%, 10%, 38.5%, and 1.5%, respectively.
2. The luciferase-expressing mRNA stock was diluted to 0.3 mg/mL with pH 4 citrate buffer to obtain an aqueous phase.
3. The oil phase (ethanol phase) containing the mixture of four lipids and the aqueous phase containing mRNA were rapidly mixed in a volume ratio of 1:3 to obtain TMF107 with a weight ratio of total lipids to mRNA of 34.9:1. The sample was then concentrated with an ultrafiltration tube having a molecular weight cutoff of 100 kd, washed with PBS buffer with a pH of 7.4 and filtered, and finally the filtrate was buffer-exchanged with 120 mg/mL sucrose PBS buffer to obtain a final sample.

Lipid nanoparticles TMF101-106 and TMF108-109 were prepared in a similar manner to lipid nanoparticles TMF100 and TMF107 using the corresponding starting materials. The details of the lipid nanoparticle components are shown in Table 2.

**Table 2: List of lipid nanoparticle components prepared from representative GOLD lipid compounds**

| | Cationic lipid | Helper lipid | | | Lipid compound of the present disclosure |
|---|---|---|---|---|---|
| TMF7 | DODMA 37% | DSPC 7.5% | Cholesterol 34.2% | PEG2000-DMG 1.3% | Compound 40 20% |
| TMF8 | DODMA 37% | DSPC 7.5% | Cholesterol 34.2% | PEG2000-DMG 1.3% | Compound 44 20% |
| TMF9 | DODMA 37% | DSPC 7.5% | Cholesterol 34.2% | PEG2000-DMG 1.3% | Compound 70 20% |
| TMF100 | DODMA 50% | DSPC 10% | - | PEG2000-DMG 1.5% | Compound 40 38.5% |
| TMF101 | DODMA 50% | DSPC 10% | - | PEG2000-DMG 1.5% | Compound 44 38.5% |
| TMF102 | DODMA 50% | DSPC 10% | - | PEG2000-DMG 1.5% | Compound 48 38.5% |
| TMF103 | DODMA 50% | DSPC 10% | - | PEG2000-DMG 1.5% | Compound 70 38.5% |
| TMF104 | DODMA 50% | DSPC 10% | - | PEG2000-DMG 1.5% | Compound 130 38.5% |
| TMF105 | DODMA 50% | DSPC 10% | - | PEG2000-DMG 1.5% | Compound 131 38.5% |
| TMF106 | DODMA 50% | DSPC 10% | - | PEG2000-DMG 1.5% | Compound 132 38.5% |
| TMF107 | DODMA 50% | DSPC 10% | - | PEG2000-DMG 1.5% | Compound 134 38.5% |
| TMF108 | DODMA 50% | DSPC 10% | - | PEG2000-DMG 1.5% | Compound 135 38.5% |
| TMF109 | DODMA 50% | DSPC 10% | - | PEG2000-DMG 1.5% | Compound 136 38.5% |

### Example 7: Detection of Lipid Nanoparticles

The size and polydispersity index (PDI) of the lipid nanoparticles were determined using NanoBrook 90plus PLAS (Brookhaven Instruments, US) by dynamic light scattering (DLS) technology at a side scatter angle of 90°. The determination results are shown in Tables 3 and 4. The content and encapsulation efficiency of the lipid nanoparticles were determined using a Qubit RNA HS quantitation assay kit (ThermoFisher Scientific, UK) according to the manufacturer's instructions. The determination results are shown in Tables 3 and 4.

**Table 3: Characterization data for lipid nanoparticles prepared from representative lipid compounds**

| Lipid nanoparticle No. | GOLD lipid used | Size(nm) | PDI (polydispersity index) | Encapsulation efficiency |
|---|---|---|---|---|
| TMF7 | 20% compound 40 | 127.5±1.2 | 0.080±0.011 | 82.70% |
| TMF8 | 20% compound 44 | 117.5±2.4 | 0.104±0.031 | 86.20% |
| TMF9 | 20% compound 70 | 122.0±1.8 | 0.129±0.024 | 86.30% |

**Table 4: Characterization data for lipid nanoparticles prepared from representative lipid compounds**

| Lipid nanoparticle No. | GOLD lipid | Cationic lipid | Class of component | Size(nm) | PDI (polydispersity index) | Encapsulation efficiency % |
|---|---|---|---|---|---|---|
| TMF100 | 38.5% compound 40 | 50% DODMA | IV | 127.52±1.24 | 0.080±0.011 | 84.8 |
| TMF101 | 38.5% compound 44 | 50% DODMA | IV | 117.47±2.38 | 0.104±0.031 | 85 |
| TMF102 | 38.5% compound 48 | 50% DODMA | IV | 151.08±1.58 | 0.094±0.023 | 63.3 |
| TMF103 | 38.5% compound 70 | 50% DODMA | IV | 122.03±1.81 | 0.129±0.024 | 86.6 |
| TMF104 | 38.5% compound 130 | 50% DODMA | IV | 237.90±4.20 | 0.137±0.017 | 10.8 |
| TMF105 | 38.5% compound 131 | 50% DODMA | IV | 266.92±10.99 | 0.210±0.028 | -13.4 |
| TMF106 | 38.5% compound 132 | 50% DODMA | IV | 259.43±2.07 | 0.152±0.057 | -4.9 |
| TMF107 | 38.5% compound 134 | 50% DODMA | IV | 306.67±7.53 | 0.245±0.027 | 7 |
| TMF108 | 38.5% compound 135 | 50% DODMA | IV | 211.21±4.07 | 0.133±0.045 | 28.5 |
| TMF109 | 38.5% compound 136 | 50% DODMA | IV | 232.09±2.33 | 0.107±0.022 | -10.3 |

### Example 8: Detection of Lipid Nanoparticle Distribution in Delivered Organs

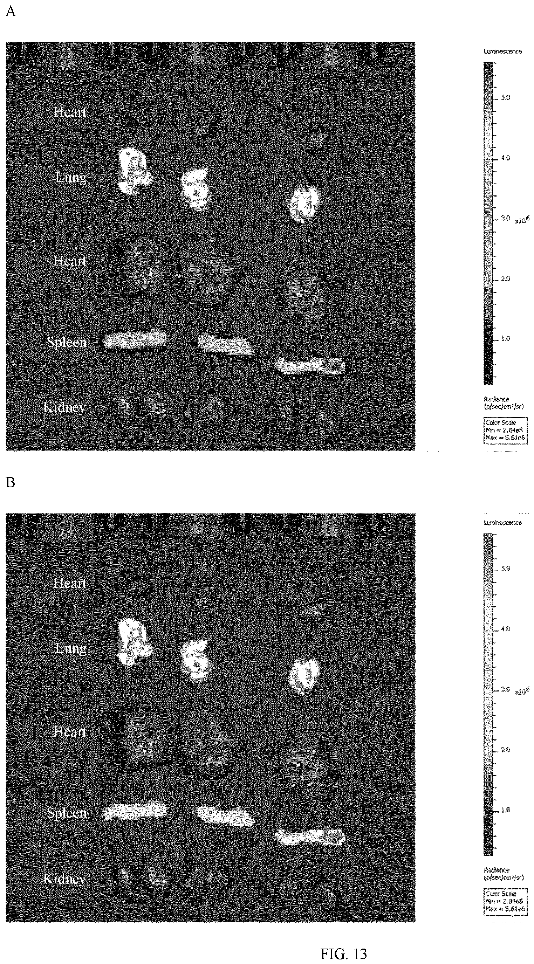

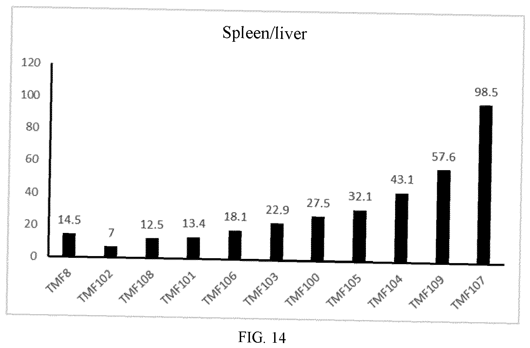

The liposomes prepared in Examples 1, 2, 3, 4, and 5 were injected at a dose of 0.5 mg/kg through the inner canthus venous plexus of the fundus to 6-8 week-old female ICR mice. 4 h after the administration, 15 mg/mL of D-luciferin potassium salt was intraperitoneally injected at a dose of 150 mg/kg. 10 min after injection of luciferase substrate, mice were placed under an in vivo imaging system (IVIS Lumina XRMS Series III, PerkinElmer) and observed for in vivo fluorescence intensity and distribution. The mice were then sacrificed, organs (heart, liver, spleen, lung and kidney) were isolated and imaged ex vivo to observe the fluorescence intensity and distribution in the different organs. The distribution of Luc-mRNA delivered by representative lipid compounds in the organs is shown in Table 5 and FIG. 13. The spleen-to-liver selectivity data are summarized in FIG. 14, and the results show that as long as the ratio of the spleen to the liver is higher than 1, the selectivity for the spleen is better and the spleen targeting effect can be achieved. The higher the ratio is, the better the spleen targeting effect is. Among them, the lipid nanoparticles TMF105, TMF104, TMF109, and TMF107 have the highest spleen-to-liver selectivity. 4 h after intravenous administration to the mice, the ratios (spleen/liver) of the mean fluorescence intensity of TMF105, TMF104, TMF109, and TMF107 in the spleen and liver are 32.1, 43.1, 57.6, and 98.5, respectively. The mean fluorescence intensity in different organs represents the delivery efficiency of the corresponding delivery system in different organs. The lipid nanoparticle of the present invention is able to successfully deliver nucleic acid molecules into the spleen and enable the expression of the nucleic acid molecules, and the delivery efficiency for the spleen is significantly higher than that for the liver and other tissues/organs.

**Table 5: Expression intensity of Luc-mRNA delivered by lipid nanoparticles prepared from representative lipid compounds.**

| Lipid nanoparticle No. | GOLD lipid used | Mean fluorescence intensity | | |
|---|---|---|---|---|
| | | Liver [p/s/cm²/sr] | Spleen [p/s/cm²/sr] | Spleen/live r |
| TMF8 | 20% compound 44 | 3.81E+05 | 5.09E+06 | 14.5 |
| TMF100 | 38.5% compound 40 | 4.00E+04 | 1.45E+06 | 27.5 |
| TMF101 | 38.5% compound 44 | 3.81E+03 | 5.09E+04 | 13.4 |
| TMF102 | 38.5% compound 48 | 1.07E+05 | 5.82E+05 | 7.0 |
| TMF103 | 38.5% compound 70 | 2.17E+05 | 4.74E+06 | 22.9 |
| TMF104 | 38.5% compound 130 | 3.96E+04 | 1.12E+06 | 43.1 |
| TMF105 | 38.5% compound 131 | 6.89E+03 | 2.21E+05 | 32.1 |
| TMF106 | 38.5% compound 132 | 1.38E+04 | 2.51E+05 | 18.1 |
| TMF107 | 38.5% compound 134 | 1.54E+04 | 1.47E+06 | 98.5 |
| TMF108 | 38.5% compound 135 | 8.81E+05 | 1.10E+07 | 12.5 |
| TMF109 | 38.5% compound 136 | 1.82E+04 | 1.05E+06 | 57.6 |

The present disclosure has been described in detail above, and it will be understood by those skilled in the art that the present disclosure should not be limited thereto. It helps to understand the mechanism and content of the present disclosure and implement it. All equivalent changes or modifications in line with the spirit of the present disclosure should fall within the scope of protection of the present disclosure.

### Industrial Practicability

The lipid compounds and their derivatives provided in this application could selectively deliver therapeutic agents / preventive agents to target organs, and their lipid nanoparticles could deliver nucleic acid components to specific organs, especially to organs outside the liver. The technical solutions in this application provided more options for the delivery of nucleic acid based drugs, gene vaccines, etc., especially for the development and application of nucleic acid preventive agents and therapeutic agents.

## Claims

1. A lipid compound of formula I or a pharmaceutically acceptable salt, a prodrug, a stereoisomer, or a deuteride thereof, 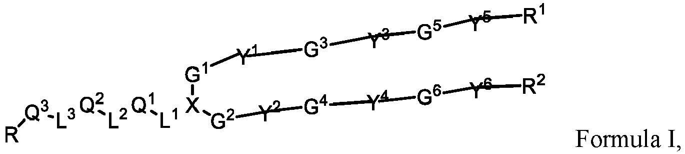
wherein L¹ is absent, -C-C-, -C=C-, -C≡C-,, -O(C=O)-, -(C=O)O-, -C(=O)-, -O(C=O)O-, -O-, - S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, - NR^{a}C(=O)O-, -NR^{a}(CH₂)ₓ-, or -(CH₂)ₓNR^{a}-; Q¹ is absent or C1-C8 linear or branched hydrocarbyl;
L² is absent, -C-C-, -C=C-, -C=C-, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-; Q² is absent or C1-C8 linear or branched hydrocarbyl;
L³ is absent, -C-C-, -C=C-, -C=C-, -O(C=O)-, -(C=O)O-, -C(=O)-, -O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or - NR^{a}C(=O)O-; Q³ is absent or C1-C8 linear or branched hydrocarbyl;
R^{a} is H, deuterium, C1-C8 linear or branched hydrocarbyl, or C1-C8 linear or branched hydrocarbyl substituted with carboxyl;
X is CorN;
R is 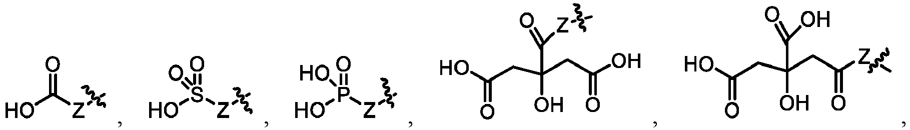 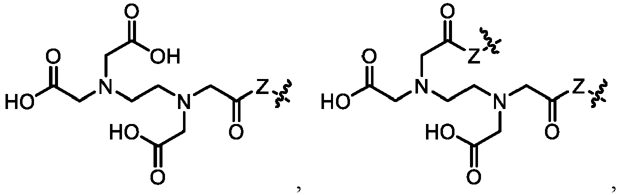 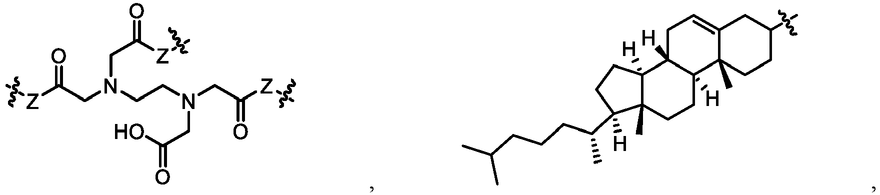 or 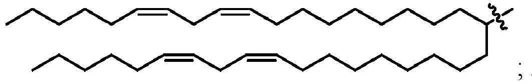 Z is C, O, NR^{b}, or S; R^{b} is H, deuterium, or C1-C8 linear or branched hydrocarbyl;
one of Y¹ and Y² is absent, -C-C-, -C=C-, -C=C-, -O(C=O)-, -C(OH)-, -(C=O)O-, -C(=O)-, - O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-, and the other one of Y¹ and Y² is absent, -C-C-, -C=C-, -C=C-, -O(C=O)-, -C(OH)-, -(C=O)O-, -C(=O)-, -O(C=O)O-, -O-, -S(O)ₓ-, -S-S-, -C(=O)S-, -SC(=O)-, - NR^{a}C(=O)-, -C(=O)NR^{a}-, -NR^{a}C(=O)NR^{a}-, -OC(=O)NR^{a}-, or -NR^{a}C(=O)O-;
G¹ and G² are each independently absent or substituted C1-C12 linear or branched hydrocarbyl;
one of Y³ and Y⁴ is absent, -C-C-, -C=C-, -C=C-, -O(C=O)-, -C(OH)-, -(C=O)O-, -C(=O)-, - O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-, and the other one of Y³ and Y⁴ is absent, -C-C-, -C=C-, -C=C-, -O(C=O)-, -C(OH)-, -(C=O)O-, -C(=O)-, -O(C=O)O-, -O-, -S(O)ₓ-, -S-S-, -C(=O)S-, -SC(=O)-, - NR^{a}C(=O)-, -C(=O)NR^{a}-, -NR^{a}C(=O)NR^{a}-, -OC(=O)NR^{a}-, or -NR^{a}C(=O)O-;
G³ and G⁴ are each independently absent or substituted C1-C12 linear or branched hydrocarbyl;
one of Y⁵ and Y⁶ is absent, -C-C-, -C=C-, -C=C-, -O(C=O)-, -C(OH)-, -(C=O)O-, -C(=O)-, - O(C=O)O-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, or -NR^{a}C(=O)O-, and the other one of Y⁵ and Y⁶ is absent, -C-C-, -C=C-, -C=C-, -O(C=O)-, -C(OH)-, -(C=O)O-, -C(=O)-, -O(C=O)O-, -O-, -S(O)ₓ-, -S-S-, -C(=O)S-, -SC(=O)-, - NR^{a}C(=O)-, -C(=O)NR^{a}-, -NR^{a}C(=O)NR^{a}-, -OC(=O)NR^{a}-, or -NR^{a}C(=O)O-;
R^{a} is H, deuterium, C1-C8 linear or branched hydrocarbyl, or C1-C8 linear or branched hydrocarbyl substituted with carboxyl;
G⁵ and G⁶ are each independently absent or substituted C1-C12 linear or branched hydrocarbyl;
R¹ and R² are each independently substituted C1-C24 linear or branched hydrocarbyl;
x is 0, 1, or 2.

2. A lipid compound shown in Table 1 or a pharmaceutically acceptable salt, a prodrug, a stereoisomer, or a deuteride thereof.

3. A lipid nanoparticle comprising the compound according to any one of claims 1 and 2 and one or more cationic lipids.

4. The lipid nanoparticle according to claim 3, further comprising one or more helper lipids.

5. The lipid nanoparticle according to claim 3, wherein the cationic lipids are selected from one of or a combination of more than one of a permanently cationic lipid and/or an ionizable cationic lipid.

6. The lipid nanoparticle according to claim 5, wherein the permanently cationic lipid is selected from one of or a combination of more than one of DOTAP, DODMA, DSTAP, DMTAP, DDA, and DOBAQ.

7. The lipid nanoparticle according to claim 5, wherein the ionizable cationic lipid is selected from one of or a combination of more than one of SM-102, Lipid 5, A6, DC-chol, C12-200, CKK-E12, 5A2-SC8, G0-C14, OF-2, 3060i10, OF-Deg-Lin, 92-0175, OF-C4-Deg-Lin, A18-iso5-2DC18, TT3, FTT5, BAMEA-O16B, Vc-Lipid, C14-4, Lipid 14, 4A3-Cit, and ssPalmO-Phe.

8. The lipid nanoparticle according to claim 4, wherein the helper lipids are selected from one of or a combination of more than one of a phospholipid and/or a steroid and/or a polymer conjugated lipid and/or a modified lipid.

9. The lipid nanoparticle according to claim 8, wherein the phospholipid is selected from one of or a combination of more than one of DOPE, DSPC, DPPC, DMPC, DOPC, POPC, and SM.

10. The lipid nanoparticle according to claim 8, wherein the steroid is selected from any one of or a combination of more than one of cholesterol, sitosterol, stigmasterol, and ergosterol.

11. The lipid nanoparticle according to claim 8, wherein the polymer conjugated lipid is selected from one or more of a polyethylene glycol conjugated lipid, a polylactic acid conjugated lipid, a polyamide conjugated lipid, a cationic polymer conjugated lipid, a poly-sarcosine (pSar) conjugated lipid, a poly(lactic-co-glycolic acid) (PLGA) conjugated lipid, a polyamino acid conjugated lipid, a polypeptide conjugated lipid, and a polypeptoid conjugated lipid.

12. The lipid nanoparticle according to claim 8, wherein the polyethylene glycol conjugated lipid is selected from any one of or a combination of more than one of PEG1000-DMG, PEG5000-DMG, PEG2000-DMG, and PEG2000-DSPE.

13. The lipid nanoparticle according to claim 8, wherein the modified lipid is selected from a lipid modified by any one of a small-molecule compound, a vitamin, a carbohydrate, a peptide, a protein, a nucleic acid lipopolysaccharide, an inorganic molecule or particle, and a metal ion or particle, or a combination thereof.

14. The lipid nanoparticle according to claim 4, wherein the lipid compound or the pharmaceutically acceptable salt, the prodrug or the stereoisomer thereof, the helper lipids, and the cationic lipids are in a molar ratio of (0.1-1):(0.5-2): 1.

15. A composition comprising the lipid nanoparticle according to any one of claims 3 to 14 and a therapeutic or prophylactic agent.

16. The composition according to claim 15, wherein the composition targets the following organs: lung, heart, brain, spleen, lymph node, bone, skeletal muscle, stomach, small intestine, large intestine/colon and rectum, kidney, bladder, breast, testis, ovary, uterus, thymus, brainstem, cerebellum, cerebrum, spinal cord, eye, ear, tongue, or skin, preferably spleen.

17. The composition according to claim 15, wherein the prophylactic or therapeutic agent is selected from any one of or a combination of more than one of a nucleic acid, a protein, a polypeptide, a small-molecule compound, and a cell.

18. The composition according to claim 15, wherein the lipid nanoparticle and the prophylactic or therapeutic agent are in a mass ratio of 10: 1 to 100:1.

19. The composition according to claim 15, wherein the composition has an average particle size of 20 nm to 600 nm.

20. The composition according to claim 15, wherein the composition has a polydispersity index (PDI) of 0.001 to 0.5.

21. The composition according to claim 17, wherein the nucleic acid is selected from a single-stranded DNA, a double-stranded DNA, a single-stranded RNA, a double-stranded RNA, a short isomer, a plasmid DNA, a complementary DNA/cDNA, an antisense oligonucleotide/ASO, a small interfering nucleic acid/siRNA, a small activating nucleic acid/saRNA, an asymmetric interfering nucleic acid/aiRNA, a micro nucleic acid/miRNA, a micro nucleic acid agonist/miRNA agomir, a micro nucleic acid inhibitor/miRNA antagomir, a Dicer enzyme substrate nucleic acid/dsRNA, a small hairpin nucleic acid/shRNA, a transfer RNA/tRNA, a messenger RNA/mRNA, a circular RNA/circRNA, a self-amplifying mRNA/samRNA, and an aptamer.

22. The composition according to claim 15, wherein the therapeutic or prophylactic agent comprises at least one mRNA encoding an antigen or a fragment thereof or an epitope thereof, or mRNA encoding a therapeutic protein.

23. The composition according to claim 22, wherein the mRNA is selected from a monocistronic mRNA and a polycistronic mRNA.

24. The composition according to claim 22, wherein the mRNA comprises one or more functional nucleotide analogs or chemically modified forms of nucleotides.

25. The composition according to claim 24, wherein the functional nucleotide analogs are selected from one of or a combination of more than one of a locked nucleic acid (LNA), a peptide nucleic acid (PNA), and a morpholine ring oligonucleotide nucleic acid mimic or functional analog.

26. The composition according to claim 24, wherein the chemical modifications of nucleotides are selected from one or more of the following modifications:
a modification to the backbone of a linking nucleotide in the nucleotide sequence of the nucleic acid molecule;
a modification to ribose in the nucleotide sequence of the nucleic acid molecule; and
a modification to a base in the nucleotide sequence of the nucleic acid molecule.

27. The composition according to claim 26, wherein the modification to the backbone is a phosphorothioate bond.

28. The composition according to claim 26, wherein the modification to ribose is selected from one of or a combination of more than one of 2'-fluororibose, ribose, 2'-deoxyribose, arabinose, and hexose.

29. The composition according to claim 26, wherein the chemical modifications of nucleotides are selected from one of or a combination of more than one of 5-methylcytosine, pseudouridine, 1-methylpseudouridine, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

30. The composition according to claim 22, wherein the antigen is a pathogenic antigen.

31. The composition according to claim 30, wherein the pathogenic antigen is selected from any one of or a combination of more than one of a tumor-associated antigen and a pathogenic microbial antigen.

32. Use of the composition according to any one of claims 15 to 31 in the preparation of a medicament.

33. A medicament comprising the composition according to any one of claims 15 to 31 and a pharmaceutically acceptable auxiliary material.
